# EUROPEAN PATENT APPLICATION

(11) **EP 4 538 286 A1**
(43) Date of publication of application: **16.04.2025**
(21) Application number: 23834646.4
(22) Date of filing: 26.06.2023
(51) Int. Cl.: C07K 14/705, A61K 38/17, A61P 35/00

(54) **CD80 MUTANT POLYPEPTIDE AND USE THEREOF**

(30) Priority: 05.07.2022 CN 202210782606
(71) Applicant: Adlai Nortye Biopharma Co., Ltd., Yuhang District Hangzhou Zhejiang 311121 (CN)
(72) Inventor: HE, Nanhai, Hangzhou, Zhejiang 311121 (CN); WANG, Youping, Hangzhou, Zhejiang 311121 (CN); CHEN, Wanwan, Hangzhou, Zhejiang 311121 (CN); ZHU, Kun, Hangzhou, Zhejiang 311121 (CN)
(74) Representative: Michalski Hüttermann & Partner Patentanwälte mbB
(86) International application number: PCT/CN2023/102271
(87) International publication number: WO 2024/007872

(57) **Abstract**

This invention relates to the field of cancer and immunotherapy. Specifically, it involves CD80 IgV mutant polypeptides, CD80 ECD mutant polypeptides, CD80 mutant fusion polypeptide complexes containing the mutant polypeptides, and nucleic acid molecules for expressing the mutant fusion polypeptides as fusion proteins. The invention also covers methods of using the mutant polypeptides, fusion polypeptide complexes, or nucleic acid molecules to induce or enhance immunity, as well as methods for treating diseases such as infection and cancer.

## Description

### Technical Field

The present invention relates to the field of cancer and immunotherapy, and in particular, the present invention relates to a CD80 IgV mutant polypeptide, a CD80 ECD mutant polypeptide, a CD80 mutant fusion polypeptide complex comprising the mutant polypeptide, and a nucleotide molecule expressing the mutant fusion polypeptide complex as a fusion protein. The present invention also relates to methods for inducing or enhancing immunity and methods for treating or preventing diseases such as infection and cancer using the mutant polypeptides, fusion polypeptide complexes or nucleotide molecules.

### Background Art

CD80 protein is a type of immune-related molecule that stimulates immune response. It mainly realizes its function by regulating T cell immune response, including participating in immune defense, immune tolerance and immune tissue damage, etc. CD80 mainly exerts immunomodulatory effects to regulate immune response by binding to its ligands CD28, CTLA4 and PDL1. Changing the affinity between CD80 and ligands can regulate CD80-mediated related functions, especially enhancing the body's immune response function and strengthening the control of diseases including tumors, infections, etc.

As one of the member proteins of the B7 molecule family protein that has co-stimulatory activation of T cells, the mature CD80 molecule is composed of an extracellular domain (ECD), a transmembrane domain and an intracellular domain, wherein the extracellular domain (ECD) is the key region for these molecules to bind to the corresponding receptors on T cells, and the CD80 extracellular domain contains an immunoglobulin variable-like V (IgV) region and an immunoglobulin constant-like C2 (IgC2) region, wherein the IgV domain is the key domain directly involved in its receptor binding; the CD80 extracellular domain IgV can bind to CD28 to participate in inducing T cell activation, proliferation and effector function. CTLA4 can also bind to the IgV region of CD80 to participate in immunosuppressive regulation.

In the immune response regulation reaction, the activation regulation of T cells is regulated by the binding of co-stimulatory signal receptors such as CD28 and the corresponding ligands CD80/CD86 on the APC surface. However, CD80 and CD86 are usually not expressed or expressed at a low level in the tumor microenvironment, resulting in the inability of T cells to be effectively activated. This is also one of the key mechanisms of tumor immune escape.

In addition, CTLA4 competes with CD28 for binding to CD80/CD86 and exhibits higher affinity characteristics, resulting in CD28 being unable to effectively receive immune stimulation signals mediated by CD80/CD86, thereby making T cells unable to be effectively activated or immunosuppressed. Moreover, the binding of PDL1 to CD80 weakens the CD80-mediated immune activation effect and interferes with the normal activation of T cells, thereby inhibiting the related immune response.

Therefore, there is a need in the art for a CD80 mutant polypeptide that improves the binding activity of CD80 to its ligand, so that it can effectively stimulate T cells and enhance the immune response, thereby treating or preventing diseases such as infections and tumors caused by the inhibition of T cell function.

### Summary of the invention

The inventors of the present application unexpectedly discovered that a CD80 mutant polypeptide formed by innovatively modifying the CD80 IgV domain can regulate the binding activity of CD80 with its ligand, so that the CD80 mutant polypeptide can effectively excite T cells and enhance the immune response, thereby achieving the treatment or prevention of diseases such as infections and tumors caused by the inhibition of T cell function.

In a first aspect, the present invention provides a CD80 IgV mutant polypeptide, which, relative to the wild-type human CD80 IgV polypeptide sequence SEQ ID NO: 45, comprises one or more amino acid mutations selected from the group consisting of T13K, A26M, A26C, E35N, E35S, E35Q, E35T, M42L, M47Q, M47H, M47K, M47T, 169Y, I69L, I69W, A71E, A71N, A71Q, A71E, V83L, L85N, and L85D.

VIHVTKEVKEVATLSCGHNVSVEELAQTRIYWQKEKKMVLTMMSGDMNIWPEYKNRTIFD ITNNLSIVILALRPSDEGTYECVVLKYEKDAFKREHLAEVTLSVKAD (SEQ ID NO: 45).

In some embodiments, the CD80 IgV mutant polypeptide comprises a combination of two or more of the amino acid mutations.

In some embodiments, the combination of two or more of the amino acid mutations is selected from E35N/A71E (SEQ ID NO: 46), E35N/A71N (SEQ ID NO: 47), E35N/A71Q (SEQ ID NO: 48), E35N/A71T (SEQ ID NO: 49), E35S/A71E (SEQ ID NO: 50), E35S/A71N (SEQ ID NO: 51), E35S/A71Q (SEQ ID NO: 52), E35S/A71T (SEQ ID NO: 53), E35Q/A71E (SEQ ID NO: 54), E35Q/A71N (SEQ ID NO: 55), E35Q/A71Q (SEQ ID NO: 56), E35Q/A71T (SEQ ID NO: 57), E35T/A71E (SEQ ID NO: 58), E35T/A71N (SEQ ID NO: 59), E35T/A71Q (SEQ ID NO: 60), E35T/A71T (SEQ ID NO: 61), M47A/I69Y/A71E/V83L (SEQ ID NO: 62), M47A/I69W/A71E/V83L (SEQ ID NO: 63), M47A/I69Y/A71E (SEQ ID NO: 64), M47A/I69W/A71E (SEQ ID NO: 65), I69Y/A71E/V83L (SEQ ID NO: 66), I69W/A71E/V83L (SEQ ID NO: 67), I69Y/A71E (SEQ ID NO: 68), I69W/A71E (SEQ ID NO: 69), H18W/A26M/A71E/L85N (SEQ ID NO: 70), H18W/A26M/A71E/L85D (SEQ ID NO: 71), H18W/A26C/A71E/L85N (SEQ ID NO: 72), H18W/A26C/A71E/L85D (SEQ ID NO: 73), A26M/A71E/L85N (SEQ ID NO: 74), A26M/A71E/L85D (SEQ ID NO: 75), A26C/A71E/L85D (SEQ ID NO: 76), A26C/A71E/L85N (SEQ ID NO: 77), I69L/A71E/V83L (SEQ ID NO: 78), H18W/A26V/E35N/A71E/L85N (SEQ ID NO: 79), T13K/M42L/M47H/A71E (SEQ ID NO: 80), T13K/M42L/M47Q/A71E (SEQ ID NO: 81), T13K/M42L/A71E (SEQ ID NO: 82), T13K/M42T/M47H/A71E (SEQ ID NO: 83), T13K/M42T/M47T/A71E (SEQ ID NO: 84), T13K/M42T/A71E (SEQ ID NO: 85), M47H/169Y/A71E/V83L (SEQ ID NO: 86), M47Q/I69Y/A71E/V83L (SEQ ID NO: 87) and M47K/I69Y/A71E/V83L (SEQ ID NO: 88).

In a second aspect, the present invention provides a CD80 ECD mutant polypeptide, which, relative to the wild-type human CD80 ECD polypeptide sequence SEQ ID NO: 1, comprises one or more amino acid mutations selected from the group consisting of T13K, A26M, A26C, E35N, E35S, E35Q, E35T, M42L, M47Q, M47H, M47K, M47T, 169Y, I69L, I69W, A71E, A71N, A71Q, A71E, V83L, L85N, and L85D.

VIHVTKEVKEVATLSCGHNVSVEELAQTRIYWQKEKKMVLTMMSGDMNIWPEYKNRTIFD ITNNLSIVILALRPSDEGTYECVVLKYEKDAFKREHLAEVTLSVKADFPTPSISDFEIPTSNIR RIICSTSGGFPEPHLSWLENGEELNAINTTVSQDPETELYAVSSKLDFNMTTNHSFMCLIKYG HLRVNQTFNWNTTKQEHFPDN (SEQ ID NO: 1).

In some embodiments, the CD80 ECD mutant polypeptide comprises a combination of two or more of the amino acid mutations.

In some embodiments, the combination of two or more of the amino acid mutations is selected from E35N/A71E (SEQ ID NO: 2), E35N/A71N (SEQ ID NO: 3), E35N/A71Q (SEQ ID NO: 4), E35N/A71T (SEQ ID NO: 5), E35S/A71E (SEQ ID NO: 6), E35S/A71N (SEQ ID NO: 7), E35S/A71Q (SEQ ID NO: 8), E35S/A71T (SEQ ID NO: 9), E35Q/A71E (SEQ ID NO: 10), E35Q/A71N (SEQ ID NO: 11), E35Q/A71Q (SEQ ID NO: 12), E35Q/A71T (SEQ ID NO: 13), E35T/A71E (SEQ ID NO: 14), E35T/A71N (SEQ ID NO: 15), E35T/A71Q (SEQ ID NO: 16), E35T/A71T (SEQ ID NO: 17), M47A/I69Y/A71E/V83L (SEQ ID NO: 18), M47A/I69W/A71E/V83L (SEQ ID NO: 19), M47A/I69Y/A71E (SEQ ID NO: 20), M47A/I69W/A71E (SEQ ID NO: 21), I69Y/A71E/V83L (SEQ ID NO: 22), I69W/A71E/V83L (SEQ ID NO: 23), I69Y/A71E (SEQ ID NO: 24), I69W/A71E (SEQ ID NO: 25), H18W/A26M/A71E/L85N (SEQ ID NO: 26), H18W/A26M/A71E/L85D (SEQ ID NO: 27), H18W/A26C/A71E/L85N (SEQ ID NO: 28), H18W/A26C/A71E/L85D (SEQ ID NO: 29), A26M/A71E/L85N (SEQ ID NO: 30), A26M/A71E/L85D (SEQ ID NO: 31), A26C/A71E/L85D (SEQ ID NO: 32), A26C/A71E/L85N (SEQ ID NO: 33), I69L/A71E/V83L (SEQ ID NO: 34), H18W/A26V/E35N/A71E/L85N (SEQ ID NO: 35), T13K/M42L/M47H/A71E (SEQ ID NO: 36), T13K/M42L/M47Q/A71E (SEQ ID NO: 37), T13K/M42L/A71E (SEQ ID NO: 38), T13K/M42T/M47H/A71E (SEQ ID NO: 39), T13K/M42T/M47T/A71E (SEQ ID NO: 40), T13K/M42T/A71E (SEQ ID NO: 41), M47H/169Y/A71E/V83L (SEQ ID NO: 42), M47Q/I69Y/A71E/V83L (SEQ ID NO: 43) and M47K/I69Y/A71E/V83L (SEQ ID NO: 44).

In a third aspect, the present invention provides a CD80 mutant fusion polypeptide complex, which comprises the CD80 IgV mutant polypeptide described in the first aspect or the CD80 ECD mutant polypeptide described in the second aspect, and a second domain.

In some embodiments, the CD80 IgV mutant polypeptide or the CD80 ECD mutant polypeptide is covalently linked to the second domain with or without a linker peptide.

In some embodiments, the second domain is an antibody or an antigen-binding fragment thereof.

In some embodiments, the antibody is selected from an immunoglobulin IgG antibody, a recombinant antibody, a chimeric antibody, a heavy chain antibody, a single domain antibody and/or a bispecific antibody.

In some embodiments, the immunoglobulin IgG antibody is selected from human IgG1, human IgG2, human IgG3, human IgG4, mouse IgG1, mouse IgG2a, mouse IgG2b or mouse IgG3.

In some embodiments, the antigen binding fragment is selected from Fab, Fab', Fv, F(ab)2, F(ab')2, scFv, VHH, di-scFv and/or dAb.

In some embodiments, the antibody or antigen-binding fragment thereof specifically targets one or more antigens selected from the group consisting of PD-L1, PD-L2, PD-1, OX40, 4-1BB, ICOS, TIGIT, CTLA4, LAG3, CD3, VEGF, VEGFR, CD47, HGF, Trop2, EpCAM, CCR8, CCR4, CCR5, GPRC5D, BCMA, CD19, CD20, HER-2 neu, DLL1, HER-3, HER-4, EGFR, PSMA, CEA, MUC-1 (mucin), MUC2, MUC3, MUC4, MUC5AC, MUC5B, MUC7, CD123, CD33, CD30, CD38, NKG2A, Nkp36 and/or Tim3.

In some embodiments, the antibody or antigen-binding fragment thereof specifically targets PD-L1, PD-1, TIGIT, CTLA4, LAG3 or CD3.

In some embodiments, the antibody or antigen-binding fragment thereof specifically targets PD-L1 or PD-1.

In some embodiments, the antibody or antigen-binding fragment thereof specifically targets LAG3.

In some embodiments, the antibody or antigen-binding fragment thereof specifically targets CD3.

In some embodiments, the antibody or antigen-binding fragment thereof targeting PD-L1 is Sugemalimab antibody or antigen-binding fragment thereof.

In some embodiments, the antibody or antigen-binding fragment thereof targeting LAG-3 is Relatlimab antibody or antigen-binding fragment thereof.

In some embodiments, the antibody or antigen-binding fragment thereof targeting CD3 is a CD3B219 antibody or an antigen-binding fragment thereof.

In some embodiments, the antibody or antigen-binding fragment thereof comprises a heavy chain, and the CD80 IgV mutant polypeptide or CD80 ECD mutant polypeptide is covalently linked to the heavy chain with or without a linker peptide.

In some embodiments, the C-terminus of the CD80 IgV mutant polypeptide or CD80 ECD mutant polypeptide is covalently linked to the N-terminus of the heavy chain with or without a linker peptide.

In some embodiments, the heavy chain C-terminus is covalently linked to the N-terminus of the CD80 IgV mutant polypeptide or CD80 ECD mutant polypeptide with or without a linker peptide.

In some embodiments, the antibody or antigen-binding fragment thereof comprises a light chain, and the CD80 IgV mutant polypeptide or CD80 ECD mutant polypeptide is covalently linked to the light chain with or without a linker peptide.

In some embodiments, the C-terminus of the CD80 IgV mutant polypeptide or CD80 ECD mutant polypeptide is covalently linked to the N-terminus of the light chain with or without a linker peptide.

In some embodiments, the C-terminus of the light chain is covalently linked to the N-terminus of the CD80 IgV mutant polypeptide or the CD80 ECD mutant polypeptide with or without a linker peptide.

In some embodiments, the CD80 mutant fusion polypeptide complex comprises a first polypeptide and a second polypeptide, and the second polypeptide may be present or absent.

In some embodiments, the first polypeptide comprises a heavy chain of an antibody or an antigen-binding fragment thereof.

In some embodiments, the first polypeptide is composed of a CD80 IgV mutant polypeptide or a CD80 ECD mutant polypeptide, a linker peptide, and a heavy chain of an antibody or an antigen-binding fragment thereof covalently linked in sequence from the N-terminus to the C-terminus of the polypeptide.

In some embodiments, the first polypeptide is composed of a heavy chain of an antibody or its antigen-binding fragment, a linker peptide, a CD80 IgV mutant polypeptide or a CD80 ECD mutant polypeptide covalently linked in sequence from the N-terminus to the C-terminus of the polypeptide.

In some embodiments, the second polypeptide comprises a light chain of an antibody or an antigen-binding fragment thereof.

In some embodiments, the second polypeptide is composed of a CD80 IgV mutant polypeptide or a CD80 ECD mutant polypeptide, a linker peptide, or a light chain of an antibody or an antigen-binding fragment thereof covalently linked in sequence from the N-terminus to the C-terminus of the polypeptide.

In some embodiments, the second polypeptide is composed of a light chain of an antibody or its antigen-binding fragment, a linker peptide, a CD80 IgV mutant polypeptide or a CD80 ECD mutant polypeptide covalently linked in sequence from the N-terminus to the C-terminus of the polypeptide.

In some embodiments, the first polypeptide is selected from E35N/A71E (SEQ ID NO: 97), E35N/A71N (SEQ ID NO: 98), E35N/A71Q (SEQ ID NO: 99), E35N/A71T (SEQ ID NO: 100), E35S/A71E (SEQ ID NO: 101), E35S/A71N (SEQ ID NO: 102), E35S/A71Q (SEQ ID NO: 103), E35S/A71T (SEQ ID NO: 104), E35Q/A71E (SEQ ID NO: 105), E35Q/A71N (SEQ ID NO: 106), E35Q/A71Q (SEQ ID NO: 107), E35Q/A71T (SEQ ID NO: 108), E35T/A71E (SEQ ID NO: 109), E35T/A71N (SEQ ID NO: 110), E35T/A71Q (SEQ ID NO: 112), E35T/A71T (SEQ ID NO: 113), M47A/I69Y/A71E/V83L (SEQ ID NO: 114), M47A/I69W/A71E/V83L (SEQ ID NO: 115), M47A/I69Y/A71E (SEQ ID NO: 116), M47A/I69W/A71E (SEQ ID NO: 117), I69Y/A71E/V83L (SEQ ID NO: 118), I69W/A71E/V83L (SEQ ID NO: 119), I69Y/A71E (SEQ ID NO: 120), I69W/A71E (SEQ ID NO: 121), H18W/A26M/A71E/L85N (SEQ ID NO: 122), H18W/A26M/A71E/L85D (SEQ ID NO: 123), H18W/A26C/A71E/L85N (SEQ ID NO: 124), H18W/A26C/A71E/L85D (SEQ ID NO: 125), A26M/A71E/L85N (SEQ ID NO: 126), A26M/A71E/L85D (SEQ ID NO: 127), A26C/A71E/L85D (SEQ ID NO: 128), A26C/A71E/L85N (SEQ ID NO: 129), I69L/A71E/V83L (SEQ ID NO: 130), H18W/A26V/E35N/A71E/L85N (SEQ ID NO: 131), T13K/M42L/M47H/A71E (SEQ ID NO: 132), T13K/M42L/M47Q/A71E (SEQ ID NO: 133), T13K/M42L/A71E (SEQ ID NO: 134), T13K/M42T/M47H/A71E (SEQ ID NO: 135), T13K/M42T/M47T/A71E (SEQ ID NO: 136), T13K/M42T/A71E (SEQ ID NO: 136), M47H/I69Y/A71E/V83L (SEQ ID NO: 137), M47Q/I69Y/A71E/V83L (SEQ ID NO: 138) or M47K/I69Y/A71E/V83L (SEQ ID NO: 139).

Alternatively, in some embodiments, the first polypeptide is selected from E35N/A71E (SEQ ID NO: 142), E35N/A71N (SEQ ID NO: 143), E35N/A71Q (SEQ ID NO: 144), E35N/A71T (SEQ ID NO: 145), E35S/A71E (SEQ ID NO: 146), E35S/A71N (SEQ ID NO: 147), E35S/A71Q (SEQ ID NO: 148), E35S/A71T (SEQ ID NO: 149), E35Q/A71E (SEQ ID NO: 150), E35Q/A71N (SEQ ID NO: 151), E35Q/A71Q (SEQ ID NO: 152), E35Q/A71T (SEQ ID NO: 153), E35T/A71E (SEQ ID NO: 154), E35T/A71N (SEQ ID NO: 155), E35T/A71Q (SEQ ID NO: 156) or E35T/A71T (SEQ ID NO: 157).

Alternatively, in some embodiments, the first polypeptide is selected from E35N/A71E (SEQ ID NO: 192), E35N/A71N (SEQ ID NO: 193), E35N/A71Q (SEQ ID NO: 194) or E35N/A71T (SEQ ID NO: 195).

Alternatively, in some embodiments, the first polypeptide is selected from E35N/A71E (SEQ ID NO: 198), E35N/A71N (SEQ ID NO: 199), E35N/A71Q (SEQ ID NO: 200) or E35N/A71T (SEQ ID NO: 201).

Alternatively, in some embodiments, the first polypeptide is selected from E35N/A71E (SEQ ID NO: 203), E35N/A71N (SEQ ID NO: 204), E35N/A71Q (SEQ ID NO: 205) or E35N/A71T (SEQ ID NO: 206).

Alternatively, in some embodiments, the first polypeptide is selected from E35N/A71E (SEQ ID NO: 209), E35N/A71N (SEQ ID NO: 210), E35N/A71Q (SEQ ID NO: 211) or E35N/A71T (SEQ ID NO: 212).

In some embodiments, the second polypeptide is selected from SEQ ID NO: 141, SEQ ID NO: 197 or SEQ ID NO: 208.

In some embodiments, the CD80 mutant fusion polypeptide complex further includes a third domain, which is covalently connected to the CD80 IgV mutant polypeptide described in the first aspect or the CD80 ECD mutant polypeptide described in the second aspect or the second domain described in the third aspect with or without a linker peptide.

In some embodiments, the third domain is an antibody or antigen-binding fragment that is the same as or different from the second domain, or the third domain is a functional protein or an active fragment thereof.

In some embodiments, when the third domain is a functional protein or an active fragment thereof, the functional protein or the active fragment thereof can activate an immune response.

In some embodiments, the second domain includes an antibody heavy chain, and the antibody heavy chain of the second domain is connected to the third domain with or without a linker peptide.

In some embodiments, the heavy chain of the second domain is connected to the N-terminus of the third domain with or without a linker peptide.

In some embodiments, the heavy chain of the second domain is connected to the C-terminus of the third domain with or without a linker peptide.

In some embodiments, the second domain includes an antibody light chain, and the antibody light chain of the second domain is connected to the third domain with or without a linker peptide.

In some embodiments, the light chain of the second domain is connected to the N-terminus of the third domain with or without a linker peptide.

In some embodiments, the light chain of the second domain is connected to the C-terminus of the third domain with or without a linker peptide.

In some embodiments, the third domain is connected to the CD80 IgV mutant polypeptide described in the first aspect or the CD80 ECD mutant polypeptide described in the second aspect with or without a linker peptide.

In some embodiments, the CD80 mutant fusion polypeptide complex includes a first polypeptide and a second polypeptide, and the second polypeptide may be present or absent.

In some embodiments, the first polypeptide includes the heavy chain of the second domain.

In some embodiments, the first polypeptide is composed of a CD80 IgV mutant polypeptide or a CD80 ECD mutant polypeptide, a linker peptide, a heavy chain of an antibody or its antigen-binding fragment, a linker peptide, and a third domain covalently linked in sequence from the N-terminus to the C-terminus, wherein the linker peptides may be the same or different and may exist or not exist independently of each other.

In some embodiments, the first polypeptide is composed of a CD80 IgV mutant polypeptide or a CD80 ECD mutant polypeptide, a linker peptide, a heavy chain of an antibody or its antigen-binding fragment, a linker peptide, and a third domain covalently linked in sequence from the C-terminus to the N-terminus, wherein the linker peptides may be the same or different and may exist or not exist independently of each other.

In some embodiments, the second polypeptide includes a light chain of the second domain.

In some embodiments, the second polypeptide is composed of a CD80 IgV mutant polypeptide or a CD80 ECD mutant polypeptide, a linker peptide, a heavy chain of an antibody or its antigen-binding fragment, a linker peptide, and a third domain covalently connected in sequence from N-terminus to C-terminus, wherein the linker peptides may be the same or different and may exist or not exist independently of each other.

In some embodiments, the second polypeptide is composed of a CD80 IgV mutant polypeptide or a CD80 ECD mutant polypeptide, a linker peptide, a heavy chain of an antibody or its antigen-binding fragment, a linker peptide, and a third domain covalently connected in sequence from the C-terminus to the N-terminus, wherein the linker peptides may be the same or different and may exist or not exist independently of each other.

In some embodiments, the third domain includes LAG-3 or an active fragment thereof.

In some embodiments, the second domain specifically targets PD-1 or PD-L1.

In some embodiments, the second domain is sugemalimab or a functional fragment thereof.

In some embodiments, the first polypeptide comprises a sequence selected from the group consisting of: SEQ ID NO: 293, SEQ ID NO: 295.

In some embodiments, the second polypeptide comprises a sequence selected from the group consisting of: SEQ ID NO: 141.

In some embodiments, the second domain is an immunoglobulin Fc domain.

In some embodiments, the Fc domain is selected from a human IgG1 Fc domain, a human IgG2 Fc domain, a human IgG3 Fc domain, a human IgG4 Fc domain, a mouse IgG1 Fc domain, a mouse IgG2a Fc domain, a mouse IgG2b Fc domain or a mouse IgG3 Fc domain.

In some embodiments, the CD80 IgV mutant polypeptide or CD80 ECD mutant polypeptide is covalently linked to the N-terminus of the immunoglobulin Fc domain with or without a linker peptide.

In some embodiments, the amino acid sequence of the CD80 mutant fusion polypeptide complex is selected from E35N/A71E (SEQ ID NO: 158), E35N/A71N (SEQ ID NO: 159), E35N/A71Q (SEQ ID NO: 160), E35N/A71T (SEQ ID NO: 161), E35S/A71E (SEQ ID NO: 162), E35S/A71N (SEQ ID NO: 163), E35S/A71Q (SEQ ID NO: 164), E35S/A71T (SEQ ID NO: 165), E35Q/A71E (SEQ ID NO: 166), E35Q/A71N (SEQ ID NO: 167), E35Q/A71Q (SEQ ID NO: 168), E35Q/A71T (SEQ ID NO: 169), E35T/A71E (SEQ ID NO: 170), E35T/A71N (SEQ ID NO: 171), E35T/A71Q (SEQ ID NO: 172) or E35T/A71T (SEQ ID NO: 173).

In some embodiments, the C-terminus of the immunoglobulin Fc domain is covalently linked to the CD80 IgV mutant polypeptide or the CD80 ECD mutant polypeptide with or without a linker peptide.

In some embodiments, wherein when a linker peptide is present, the linker peptide is selected from one or more of GGGGS (SEQ ID NO: 92), GGGGSGGGGS (SEQ ID NO: 93), GGGGSGGGGSGGGGS (SEQ ID NO: 94), GGGGSGGGGSGGGGSGGGGS (SEQ ID NO: 95) and GGGGSGGGS (SEQ ID NO: 96).

In a fourth aspect, the present invention provides an immunoconjugate comprising the CD80 IgV mutant polypeptide as described in the first aspect, the CD80 ECD mutant polypeptide as described in the second aspect, or the CD80 mutant fusion polypeptide complex as described in the third aspect.

In a fifth aspect, the present invention provides a nucleotide molecule encoding the CD80 IgV mutant polypeptide as described in the first aspect, the CD80 ECD mutant polypeptide as described in the second aspect, or the CD80 mutant fusion polypeptide complex as described in the third aspect.

In a sixth aspect, the present invention provides a vector comprising the nucleotide molecule described in the fifth aspect.

In the seventh aspect, the present invention provides a host cell, comprising the CD80 IgV mutant polypeptide as described in the first aspect, the CD80 ECD mutant polypeptide as described in the second aspect, the CD80 mutant fusion polypeptide complex as described in the third aspect, the nucleotide molecule as described in the fifth aspect and/or the vector as described in the sixth aspect.

In an eighth aspect, the present invention provides a composition comprising the CD80 IgV mutant polypeptide as described in the first aspect, the CD80 ECD mutant polypeptide as described in the second aspect, the CD80 mutant fusion polypeptide complex as described in the third aspect, the immunoconjugate as described in the fourth aspect, the nucleotide molecule as described in the fifth aspect or the vector as described in the sixth aspect and a pharmaceutically acceptable carrier.

In the ninth aspect, the present invention provides a method for preparing the CD80 IgV mutant polypeptide as described in the first aspect, the CD80 ECD mutant polypeptide as described in the second aspect, or the CD80 mutant fusion polypeptide complex as described in the third aspect, comprising culturing the host cell as described in the seventh aspect to express the CD80 IgV mutant polypeptide, CD80 ECD mutant polypeptide and/or CD80 mutant fusion polypeptide complex.

In the tenth aspect, the present invention provides a method for regulating T cell activity, comprising contacting the CD80 IgV mutant polypeptide as described in the first aspect, the CD80 ECD mutant polypeptide as described in the second aspect, or the CD80 mutant fusion polypeptide complex as described in the third aspect and/or the composition as described in the eighth aspect with T cells to stimulate their activation.

In the eleventh aspect, the present invention provides a method for inhibiting the growth or proliferation of tumor cells, comprising administering the CD80 IgV mutant polypeptide as described in the first aspect, the CD80 ECD mutant polypeptide as described in the second aspect, the CD80 mutant fusion polypeptide complex as described in the third aspect, the immunoconjugate as described in the fourth aspect, the nucleotide molecule as described in the fifth aspect, the vector as described in the sixth aspect, the host cell as described in the seventh aspect and/or the composition as described in the eighth aspect.

In the twelfth aspect, the present invention provides the use of the CD80 IgV mutant polypeptide as described in the first aspect, the CD80 ECD mutant polypeptide as described in the second aspect, the CD80 mutant fusion polypeptide complex as described in the third aspect, the immunoconjugate as described in the fourth aspect, the nucleotide molecule as described in the fifth aspect, the vector as described in the sixth aspect, the host cell as described in the seventh aspect and/or the composition as described in the eighth aspect in the preparation of a medicament for preventing, improving and/or treating tumors or cancer.

The following specific embodiments and examples describe embodiments of the present invention in detail. It will be understood that the present invention is not limited to the specific embodiments described herein and is therefore susceptible to numerous variations and modifications. Those skilled in the art will recognize that such variations and modifications are within the scope and spirit of the invention.

### BRIEF DESCRIPTION OF THE DRAWINGS

Figure 1A-Figure 1O show the expression of CD80 mutant fusion polypeptide complex detected by SDS-PAGE.
Figures 2A-2D exemplarily show the use of ELISA to detect the binding of CD80 mutant fusion polypeptide complexes to human PDL1 protein.
Figures 3A-3O exemplarily show the use of ELISA to detect the binding of CD80 mutant fusion polypeptide complexes to human CTLA4 protein.
Figures 4A-4O exemplarily show the use of ELISA to detect the binding of CD80 mutant fusion polypeptide complexes to human CD28 protein.
Figures 5A-5L exemplarily show that the CD80 mutant fusion polypeptide complex relieves the PD-1/PD-L1-mediated T cell activation immunosuppressive response.
Figures 6A-6D exemplarily show that the CD80 mutant fusion polypeptide complex regulates Jurkat T cell activation.
Figures 7A-7C exemplarily show that the CD80 mutant fusion polypeptide complex regulates PBMC activation.
Figures 8A-8C show that the CD80 mutant fusion polypeptide complex promotes the secretion of chemokines by antigen presenting cells.

### Embodiments

Unless otherwise defined, all scientific and technical terms used herein have the same meaning as commonly understood by one of ordinary skill in the art to which this invention belongs. In case of conflict, the definitions in this specification shall control.

The terminology used herein is for the purpose of describing particular embodiments only and is not intended to be limiting of the invention. Although only certain exemplary materials and methods are described herein, many methods and materials similar or equivalent to those described herein can be used in the practice of the present invention.

As used herein, the singular forms "a", "an" and "the" are intended to include the plural forms as well, unless the context clearly indicates otherwise. In addition, the open-ended expressions "include" and "comprise" are interpreted as also including structural components or method steps that are not mentioned, but it should be noted that the open-ended expressions also cover the situation consisting only of the described components and method steps (that is, they cover the situation of the closed expression "consisting of...").

In general, the term "about" is used herein to refer to a numerical value above and below the stated value with a modification of 5%.

The term "regulating T cell activity" refers to the effect of regulating lymphoid T cells by the CD80 mutant polypeptide and CD80 mutant fusion polypeptide complex disclosed in the present invention, and can be extended to any other related T cell regulators that affect the expression of T cell NFAT transcription factors, the secretion of cytokines such as IL-2, IFN-γ, TNFα and Granzyme B, cell proliferation and cell killing of target cells including tumor cells after treating T cells. The T cells can be extended to T cell lines and/or primary T cells including but not limited to CD4 T cells and CD4 T cell subsets Th1, Th2, Th9, Th17, TFH and/or Treg cells; CD8 T cells are also included, including but not limited to tumor tissue infiltrating CD8 T cells, effector CD8 T cells and/or immune memory CD8 T cells.

The term "anti-tumor activity" refers to any biological activity that reduces or prevents the proliferation or viability of tumor cells in vivo and/or in vitro. In certain embodiments, the anti-tumor activity is the anti-tumor effect of the CD80 mutant polypeptide and its fusion polypeptide complex described in the present invention.

The term 'CD80' refers to a polypeptide that protein encoded by the gene with the NCBI accession number Gene ID: 941, which has at least about 85% amino acid identity and exhibits binding activity to CD28 (protein encoded by the gene with NCBI accession number Gene ID: 940) and/or CTLA4 (protein encoded by the gene with NCBI accession number Gene ID: 1493) and/or PD-L1 (protein encoded by the gene with NCBI accession number Gene ID: 29126). Below is the exemplary human CD80 amino acid sequence.

VIHVTKEVKEVATLSCGHNVSVEELAQTRIYWQKEKKMVLTMMSGDMNIWPEYKNRTIFD
ITNNLSIVILALRPSDEGTYECVVLKYEKDAFKREHLAEVTLSVKADFPTPSISDFEIPTSNIR
RIICSTSGGFPEPHLSWLENGEELNAINTTVSQDPETELYAVSSKLDFNMTTNHSFMCLIKYG
HLRVNQTFNWNTTKQEHFPDNLLPSWAITLISVNGIFVICCLTYCFAPRCRERRRNERLRRES
VRPV(SEQ ID NO: 89).

The term "CD80 extracellular region" means an amino acid sequence of a domain in the extracellular region of the CD80 protein and at least about 85% amino acid identity, and a polypeptide or a fragment thereof having CD28 and/or CTLA4 binding activity. An exemplary "CD80 extracellular region amino acid sequence" (SEQ ID NO: 1). The term "CD80 IgV" refers to a polypeptide or fragment thereof having an amino acid sequence having at least about 85% amino acid identity with the IgV domain in the extracellular region of the CD80 protein and having CD28 and/or CTLA4 binding activity. An exemplary "CD80 IgV" (SEQ ID NO: 45).

The term "amino acid identity" refers to when amino acid sequences are compared and aligned (if necessary, introducing gaps) for maximum correspondence. The numerical value of the percent identity can be determined using sequence comparison software, or an algorithm, or by visual inspection, and for the comparison of the amino acid identity of two or more or mixed tandems of the second binding domain, any subunit of the second binding domain should be compared with it separately to calculate the percent identity with any subunit of the second binding domain; different algorithms and software known in the art can be used to obtain amino acid sequence alignments, such as but not limited to NCBI BLAST software.

The term 'immunoglobulin' refers to an antibody comprising amino acid fragments of antigen-specific binding regions and constant regions. The antigen-specific binding region, which is the fragment that determines the key differences in immunoglobulins, can also be referred to as the antigen-binding domain or as an 'epitope' or 'antigenic determinant.' The antigen-binding domain typically consists of the antibody heavy chain variable region (VH) and the antibody light chain variable region (VL). However, it does not necessarily include both. The antigen-binding domains of the antibodies disclosed in this invention are not limited to the conventional domains composed of VH and VL but also include any other types of antibodies, including but not limited to recombinant antibodies, single-domain antibodies, heavy chain antibodies, chimeric antibodies, bispecific antibodies, and other non-conventional antibodies and their combinations. The constant region refers to the common structural regions of immunoglobulins, including the constant regions of the antibody light chain and the antibody heavy chain.

In the present application, the term "immunoglobulin Fc domain" generally refers to the Fc fragment of one Fc fragment and two identical Fab fragments formed by papain hydrolysis of traditional antibody IgG. The Fc domain can include antibody heavy chain CH2, CH3 and hinge region fragments. The traditional Fc fragment has the function of binding to the Fc fragment receptor to mediate related biological effects, and site-specific mutation can change its binding ability to the corresponding target receptor, thus affecting its biological function, and the immunoglobulin Fc domain disclosed in this application should include but not limited to traditional Fc fragments and any other forms of Fc mutants. The following provides an exemplary human immunoglobulin IgG1 Fc domain (SEQ ID NO: 90) and Fc domain of human immunoglobulin IgG4 (SEQ ID NO: 91):

EPKSCDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPPVL
DSDGSFFLYSKLTVD KSRWQQGNVFSCSVMHEALHNHYTQKSLSLSPGK (SEQ ID NO:
90);

ESKYGPPCPSCPAPEFLGGPSVFLFPPKPKDTLMISRTPEVTCVVVDVSQEDPEVQFNWYVD
GVEVHNAKTKPREEQFNSTYRVVSVLTVLHQDWLNGKEYKCKVSNKGLPSSIEKTISKAK
GQPREPQVYTLPPSQEEMTKNQVSLTCLVKGFYPSDIAVEWESNGQPENNYKTTPPVLDSD
GSFFLYSRLTVDKSRWQEGNVFSCSVMHEALHNHYTQKSLSLSLGK(SEQ ID NO: 91).

The term 'linker peptide' refers to a short peptide chain with conformational flexibility, formed by a combination of Gly (G) and Ser (S) amino acid residues, wherein the ratio of the number of Gly amino acids to the number of Ser amino acids is ensured to be ≥1. The linker peptides disclosed in this invention can be extended to any short peptides with this characteristic. Below are exemplary linker peptide amino acid sequences, including but not limited to: GGGGS(SEQ ID NO: 92), GGGGSGGGGS (SEQ ID NO: 93), GGGGSGGGGSGGGGS (SEQ ID NO: 94), GGGGSGGGGSGGGGSGGGGS (SEQ ID NO: 95) and GGGGSGGGS (SEQ ID NO: 96).

In this invention, the term 'CD80 mutant polypeptide' refers to a CD80 ECD mutant polypeptide and/or a CD80 IgV mutant polypeptide. The term 'fusion polypeptide complex of mutant polypeptides' is also referred to in this invention as 'mutant polypeptide complex,' 'polypeptide complex,' 'fusion polypeptide complex,' or 'fusion complex.' 'CD80 mutant fusion polypeptide complex' refers to a functional fusion polypeptide complex in which a CD80 ECD mutant or CD80 IgV mutant is linked, with or without a linker peptide, to an antibody or its antigen-binding fragment, or an immunoglobulin Fc domain, or a functional polypeptide fragment. In the disclosed functional fusion polypeptide complex where the CD80 mutant is linked, with or without a linker peptide, to an antibody or its antigen-binding fragment, the antibody includes but is not limited to anti-PD-L1 antibody, anti-PD-1 antibody, anti-TIGIT antibody, anti-CTLA4 antibody, anti-CEA antibody, anti-BCMA antibody, anti-LAG3 antibody, anti-CD3 antibody, anti-Her2 antibody, anti-Her3 antibody, anti-VEGF antibody, anti-VEGFR antibody, anti-EGFR antibody, anti-c-Met antibody, anti-CD19 antibody, anti-CD20 antibody, anti-CD38 antibody, anti-TROP-2 antibody, anti-CD40 antibody, anti-4-1BB antibody, anti-CD30 antibody, etc. Any form of antibody covalently linked, with or without a linker peptide, to the CD80 ECD mutant or CD80 IgV mutant disclosed in this invention, or their combinations, should be considered within the scope of this invention.

The term "LAG3" refers to a protein that shows at least about 85% amino acid identity with the protein encoded by the gene with NCBI Gene ID: 3902, and possesses binding activity to MHCII (also known as HLA-DR), which is assembled from the α subunit (encoded by the gene with NCBI Gene ID: 3122) and β subunit (encoded by the genes with NCBI Gene IDs: 3123 or 3125), and/or FGL1 (encoded by the gene with NCBI Gene ID: 2267), as well as the ability to promote the activation and maturation of antigen-presenting cells. This may include peptides or fragments thereof.

The term "LAG3 active fragment" refers to an active fragment derived from the LAG3 protein that possesses functional characteristics of the LAG3 protein. The functional characteristics of the LAG3 protein include, but are not limited to, binding to MHCII (also known as HLA-DR) and/or FGL1, and promoting the activation and maturation of antigen-presenting cells.

The activation and maturation of antigen-presenting cells include, but are not limited to, upregulation of co-stimulatory receptor expression on antigen-presenting cells, enhanced antigen phagocytosis, increased cytokine secretion, and enhanced chemokine secretion.

The co-stimulatory receptors include but are not limited to ICOSL, CD40L, CD137L, OX40L, CD80, CD83 and CD86.

The antigen phagocytosis includes but is not limited to the effect of phagocytosis of bacteria, viruses, proteins, polysaccharides, etc.

The cytokines include but are not limited to IL-1beta, TNFa, IL-6, IL-12, etc.

The chemokines include but are not limited to CCL1, CCL2, CCL3, CCL4, CCL5, CXCL1, CXCL2, CXCL3, CXCL4, CXCL5, etc.

The inventors of the present application unexpectedly discovered that a CD80 mutant polypeptide formed by innovatively modifying the CD80 IgV domain can regulate the binding activity of CD80 with its ligand, so that the CD80 mutant polypeptide can effectively excite T cells and enhance the immune response, thereby achieving the treatment or prevention of diseases such as infections and tumors caused by the inhibition of T cell function.

On this basis, the present invention provides a class of CD80 mutant polypeptides including CD80 IgV mutant polypeptides and CD80 ECD mutant polypeptides; CD80 mutant fusion polypeptide complexes; and methods for regulating T cell immune responses and applications thereof in disease treatment.

In a first aspect, the present invention provides a CD80 IgV mutant polypeptide, which includes one or more amino acid position substitution mutations of wild-type CD80 IgV, which can be can be substituted by amino acid mutations in a human CD80 IgV polypeptide (SEQ ID NO: 45)amino acid substitution mutation, with the mutation sites selected from positions 13, 18, 26, 35, 42, 47, 69, 71, 83, 85. In some embodiments, CD80 IgV mutant polypeptide comprises two or more amino acid substitution mutations on SEQ ID NO:45, with the mutation sites selected from positions 13, 18, 26, 35, 42, 47, 69, 71, 83, 85 on SEQ ID NO:45. In some embodiments, the CD80 IgV mutant polypeptide comprises three or more amino acid substitution mutations on SEQ ID NO: 45, with the mutation sites selected from positions 13, 18, 26, 35, 42, 47, 69, 71, 83, and 85 on SEQ ID NO: 45. In some embodiments, CD80 IgV mutant polypeptide comprises four or more amino acid substitution mutations on SEQ ID NO: 45, with the mutation sites selected from positions 13, 18, 26, 35, 42, 47, 69, 71, 83, 85 on SEQ ID NO: 45. In some embodiments, CD80 IgV mutant polypeptide comprises five or more amino acid substitution mutations on SEQ ID NO: 45, with the mutation sites selected from positions 13, 18, 26, 35, 42, 47, 69, 71, 83, 85 on SEQ ID NO: 45.

In some embodiments, the mutation site of the CD80 IgV mutant polypeptide comprises SEQ ID NO: One or more of mutations T13K, A26M, A26C, E35N, E35S, E35Q, E35T, M42L, M47Q, M47H, M47K, M47T, 169Y, I69L, 169W, A71E, A71N, A71Q, A71E, V83L, L85N, and L85D at amino acid positions 45.

In some preferred embodiments, the mutation sites of the CD80 IgV mutant polypeptide comprise a combination of mutations at two or more amino acid positions on SEQ ID NO: 45. The CD80 IgV mutant polypeptide may be SEQ ID NO: 46), E35N/A71N (SEQ ID NO: 47), E35N/A71Q (SEQ ID NO: 48), E35N/A71T (SEQ ID NO: 49), E35S/A71E (SEQ ID NO: 50), E35S/A71N (SEQ ID NO: 51), E35S/A71Q (SEQ ID NO: 52), E35S/A71T (SEQ ID NO: 53), E35Q/A71E (SEQ ID NO: 54), E35Q/A71N (SEQ ID NO: 55), E35Q/A71Q (SEQ ID NO: 56), E35Q/A71T (SEQ ID NO: 57), E35T/A71E (SEQ ID NO: 58), E35T/A71N (SEQ ID NO: 59), E35T/A71Q (SEQ ID NO: 60), E35T/A71T (SEQ ID NO: 61), M47A/I69Y/A71E/V83L (SEQ ID NO: 62), M47A/I69W/A71E/V83L (SEQ ID NO: 63), M47A/I69Y/A71E (SEQ ID NO: 64), M47A/I69W/A71E (SEQ ID NO: 65), I69Y/A71E/V83L (SEQ ID NO: 66), I69W/A71E/V83L (SEQ ID NO: 67), I69Y/A71E (SEQ ID NO: 68), I69W/A71E (SEQ ID NO: 69), H18W/A26M/A71E/L85N (SEQ ID NO: 70), H18W/A26M/A71E/L85D (SEQ ID NO: 71), H18W/A26C/A71E/L85N (SEQ ID NO: 72), H18W/A26C/A71E/L85D (SEQ ID NO: 73), A26M/A71E/L85N (SEQ ID NO: 74), A26M/A71E/L85D (SEQ ID NO: 75), A26C/A71E/L85D (SEQ ID NO: 76), A26C/A71E/L85N (SEQ ID NO: 77), I69L/A71E/V83L (SEQ ID NO: 78), H18W/A26V/E35N/A71E/L85N (SEQ ID NO: 79), T13K/M42L/M47H/A71E (SEQ ID NO: 80), T13K/M42L/M47Q/A71E (SEQ ID NO: 81), T13K/M42L/A71E (SEQ ID NO: 82), T13K/M42T/M47H/A71E (SEQ ID NO: 83), T13K/M42T/M47T/A71E (SEQ ID NO: 84), T13K/M42T/A71E (SEQ ID NO: 85), M47H/169Y/A71E/V83L (SEQ ID NO: 86), M47Q/I69Y/A71E/V83L (SEQ ID NO: 87), M47K/I69Y/A71E/V83L (SEQ ID NO: 88).

In the second aspect, the present invention provides a CD80 ECD mutant polypeptide, which includes one or more amino acid substitution mutations at sites on the wild-type CD80 ECD. It can be derived from the human CD80 ECD polypeptide (SEQ ID NO: 1) amino acid substitution mutations, with mutation sites selected from the sites 13, 18, 26, 35, 42, 47, 69, 71, 83, 85 on SEQ ID NO: 1. In some embodiments, CD80 ECD mutant polypeptide comprises two or more amino acid substitution mutations on SEQ ID NO: 1, with the mutation sites selected from positions 13, 18, 26, 35, 42, 47, 69, 71, 83, 85 on SEQ ID NO: 1. In some embodiments, CD80 ECD mutant polypeptide comprises three or more amino acid substitution mutations on SEQ ID NO: 1, with the mutation sites selected from positions 13, 18, 26, 35, 42, 47, 69, 71, 83, 85 on SEQ ID NO: 1. In some embodiments, CD80 ECD mutant polypeptide comprises four or more amino acid substitution mutations on SEQ ID NO: 1, with the mutation sites selected from positions 13, 18, 26, 35, 42, 47, 69, 71, 83, 85 on SEQ ID NO: 1. In some embodiments, CD80 ECD mutant polypeptide comprises five or more amino acid substitution mutations on SEQ ID NO: 1, with the mutation sites selected from positions 13, 18, 26, 35, 42, 47, 69, 71, 83, 85 on SEQ ID NO: 1.

In some embodiments, the mutation sites of the CD80 ECD mutant polypeptide include one or more mutations at the amino acid sites on SEQ ID NO: 1, selected from T13K, A26M, A26C, E35N, E35S, E35Q, E35T, M42L, M47Q, M47H, M47K, M47T, 169Y, I69L, I69W, A71E, A71N, A71Q, A71E, V83L, L85N, L85D.

In some preferred embodiments, the mutation sites of the CD80 ECD mutant polypeptide include a combination of mutations at two or more amino acid sites on SEQ ID NO: 1. The CD80 ECD mutant polypeptide can be: E35N/A71E(SEQ ID NO: 2), E35N/A71N (SEQ ID NO: 3), E35N/A71Q (SEQ ID NO: 4), E35N/A71T (SEQ ID NO: 5), E35S/A71E (SEQ ID NO: 6), E35S/A71N (SEQ ID NO: 7), E35S/A71Q (SEQ ID NO: 8), E35S/A71T (SEQ ID NO: 9), E35Q/A71E (SEQ ID NO: 10), E35Q/A71N (SEQ ID NO: 11), E35Q/A71Q (SEQ ID NO: 12), E35Q/A71T (SEQ ID NO: 13), E35T/A71E (SEQ ID NO: 14), E35T/A71N (SEQ ID NO: 15), E35T/A71Q (SEQ ID NO: 16), E35T/A71T (SEQ ID NO: 17), M47A/I69Y/A71E/V83L (SEQ ID NO: 18), M47A/I69W/A71E/V83L (SEQ ID NO: 19), M47A/I69Y/A71E (SEQ ID NO: 20), M47A/I69W/A71E (SEQ ID NO: 21), I69Y/A71E/V83L (SEQ ID NO: 22), I69W/A71E/V83L (SEQ ID NO: 23), I69Y/A71E (SEQ ID NO: 24), I69W/A71E (SEQ ID NO: 25), H18W/A26M/A71E/L85N (SEQ ID NO: 26), H18W/A26M/A71E/L85D (SEQ ID NO: 27), H18W/A26C/A71E/L85N (SEQ ID NO: 28), H18W/A26C/A71E/L85D (SEQ ID NO: 29), A26M/A71E/L85N (SEQ ID NO: 30), A26M/A71E/L85D (SEQ ID NO: 31), A26C/A71E/L85D (SEQ ID NO: 32), A26C/A71E/L85N (SEQ ID NO: 33), I69L/A71E/V83L (SEQ ID NO: 34), H18W/A26V/E35N/A71E/L85N (SEQ ID NO: 35), T13K/M42L/M47H/A71E (SEQ ID NO: 36), T13K/M42L/M47Q/A71E (SEQ ID NO: 37), T13K/M42L/A71E (SEQ ID NO: 38), T13K/M42T/M47H/A71E (SEQ ID NO: 39), T13K/M42T/M47T/A71E (SEQ ID NO: 40), T13K/M42T/A71E (SEQ ID NO: 41), M47H/169Y/A71E/V83L (SEQ ID NO: 42), M47Q/I69Y/A71E/V83L (SEQ ID NO: 43), M47K/I69Y/A71E/V83L (SEQ ID NO: 44).

In a third aspect, the present invention provides a fusion polypeptide complex of a CD80 mutant polypeptide, which comprises the above-mentioned CD80 ECD mutant polypeptide or CD80 IgV mutant polypeptide, covalently linked to a second domain with or without a linker peptide, wherein the second domain can be an immunoglobulin Fc domain, an antibody or an antigen-binding fragment thereof, or other forms of polypeptides.

In some embodiments, the fusion polypeptide complex of the CD80 mutant polypeptide comprises the above-mentioned CD80 ECD mutant polypeptide or CD80 IgV mutant polypeptide, covalently linked to an immunoglobulin Fc domain with or without a linker peptide, wherein the Fc domain includes but is not limited to a human IgG1 Fc domain, a human IgG2 Fc domain, a human IgG3 Fc domain, a human IgG4 Fc domain, a mouse IgG1 Fc domain, a mouse IgG2a Fc domain, a mouse IgG2b Fc domain or a mouse IgG3 Fc domain.

In some embodiments, the CD80 mutant polypeptide is covalently linked to the N-terminus of the immunoglobulin Fc domain with or without a linker peptide to form a fusion polypeptide complex of the CD80 mutant polypeptide.

In some embodiments, the C-terminus of the immunoglobulin Fc domain is covalently linked to the CD80 mutant polypeptide with or without a linker peptide to form a fusion polypeptide complex of the CD80 mutant polypeptide.
In some embodiments, the fusion polypeptide complex of the CD80 mutant polypeptide comprises the above-mentioned CD80 ECD mutant polypeptide or CD80 IgV mutant polypeptide, covalently linked to an antibody or an antigen-binding fragment thereof with or without a linker peptide. In some embodiments, the antibody can be selected from immunoglobulin IgG antibodies, recombinant antibodies, chimeric antibodies, heavy chain antibodies, single domain antibodies and/or bispecific antibodies. In some embodiments, the antigen binding fragment can be selected from Fab, Fab', Fv, F(ab)2, F(ab')2, scFv, di-scFv, VHH and/or dAb.

In some embodiments, the CD80 mutant fusion polypeptide complex comprises the above-mentioned CD80 ECD mutant polypeptide or CD80 IgV mutant polypeptide, covalently linked to an immunoglobulin IgG antibody with or without a linker peptide, and the immunoglobulin IgG includes but is not limited to human IgG1, human IgG2, human IgG3, human IgG4, mouse IgG1, mouse IgG2a, mouse IgG2b or mouse IgG3.

In some preferred embodiments, the antibody or antigen-binding fragment in the fusion polypeptide complex of the above-mentioned CD80 mutant polypeptide specifically targets a protein selected from but not limited to PD-L1, PD-L2, PD-1, OX40, 4-1BB, ICOS, TIGIT, CTLA4, LAG3, CD3, VEGF, VEGFR, CD47, HGF, Trop2, EpCAM, CCR8, CCR4, CCR5, GPRC5D, BCMA, CD19, CD20, HER-2 neu, DLL1, HER-3, HER-4, EGFR, PSMA, CEA, MUC-1 (mucin), MUC2, MUC3, MUC4, MUCSAC, MUC5B, MUC7, CD123, CD33, CD30, CD38, NKG2A, Nkp36 and/or Tim3.

In a preferred embodiment, the anti-PD-L1 antibody or antigen-binding fragment is Sugemalimab antibody or an antigen-binding fragment thereof.

In a preferred embodiment, the anti-LAG-3 antibody or antigen-binding fragment is Relatlimab antibody or an antigen-binding fragment thereof.

In a preferred embodiment, the anti-CD3 antibody or antigen-binding fragment is a CD3B219 antibody or an antigen-binding fragment thereof.

Alternatively, in some embodiments, the present invention provides a fusion polypeptide complex of a CD80 mutant polypeptide, wherein the CD80 mutant polypeptide and the antibody or antigen-binding fragment are connected via a linker peptide.

In one embodiment, the present invention provides a fusion polypeptide complex of a CD80 mutant polypeptide, wherein the CD80 mutant polypeptide and the antibody or antigen-binding fragment are directly linked without a linker peptide.

In one embodiment, the antibody or antigen-binding fragment thereof comprises a heavy chain, and the CD80 mutant polypeptide is covalently linked to the heavy chain with or without a linker peptide.

In a preferred embodiment, the C-terminus of the CD80 mutant polypeptide is covalently linked to the N-terminus of the heavy chain with or without a linker peptide to form a fusion polypeptide complex of the CD80 mutant polypeptide.

In a preferred embodiment, the C-terminus of the heavy chain is covalently linked to the N-terminus of the CD80 mutant polypeptide with or without a linker peptide to form a fusion polypeptide complex of the CD80 mutant polypeptide.

In one embodiment, the antibody or antigen-binding fragment thereof comprises a light chain, and the CD80 mutant polypeptide is covalently linked to the light chain with or without a linker peptide.

In a preferred embodiment, the C-terminus of the CD80 mutant polypeptide is covalently linked to the N-terminus of the light chain with or without a linker peptide to form a fusion polypeptide complex of the CD80 mutant polypeptide.

In a preferred embodiment, the C-terminus of the light chain is covalently linked to the N-terminus of the CD80 mutant polypeptide with or without a linker peptide to form a fusion polypeptide complex of the CD80 mutant polypeptide.

In one embodiment, the CD80 mutant polypeptide is covalently linked to the N-terminus of the antibody or antigen-binding fragment thereof with or without a linker peptide.

In one embodiment, the CD80 mutant polypeptide is covalently linked to the C-terminus of the antibody or antigen-binding fragment thereof with or without a linker peptide.

In another embodiment, the CD80 mutant fusion polypeptide complex provided by the present invention includes a first polypeptide and a second polypeptide constituting the fusion polypeptide complex. It should be understood that the second polypeptide may not be present in certain specific antibodies.

In a preferred embodiment, the first polypeptide of the fusion polypeptide complex of the present invention comprises a heavy chain of an antibody or an antigen-binding fragment thereof.

In a preferred embodiment, the first polypeptide of the fusion polypeptide complex of the present invention is formed by covalently linking in sequence from the N-terminus to the C-terminus of the polypeptide a CD80 mutant polypeptide, a linker peptide, and a heavy chain of an antibody or an antigen-binding fragment thereof.

In a preferred embodiment, the first polypeptide of the polypeptide complex of the present invention is formed by covalently linking in sequence from the N-terminus to the C-terminus of the polypeptide the heavy chain of the antibody or its antigen-binding fragment, a linker peptide, and a CD80 mutant polypeptide.

In a preferred embodiment, the second polypeptide of the polypeptide complex of the present invention comprises a light chain of an antibody or an antigen-binding fragment thereof.

In a preferred embodiment, the second polypeptide of the polypeptide complex of the present invention is covalently linked in sequence from the N-terminus to the C-terminus of the polypeptide by a CD80 mutant polypeptide, a linker peptide, and a light chain of an antibody or an antigen-binding fragment thereof.

In a preferred embodiment, the second polypeptide of the polypeptide complex of the present invention is formed by covalently linking in sequence from the N-terminus to the C-terminus of the polypeptide a light chain of an antibody or an antigen-binding fragment thereof, a linker peptide, and a CD80 mutant polypeptide.

Alternatively, in some embodiments, the present invention also provides a CD80 mutant fusion polypeptide complex, comprising a CD80 mutant polypeptide, a second domain and a third domain, wherein the CD80 mutant polypeptide and the second domain are the same as described above.

In some embodiments, the third domain of the present invention is an antibody or antigen-binding fragment that is the same as or different from the second domain, or the third domain is a functional protein capable of immune activation or an active fragment thereof.

In some embodiments, the second domain of the present invention comprises a heavy chain, and the third domain of the present invention is connected to the heavy chain of the second domain with or without a linker peptide.

In a specific embodiment, the heavy chain of the second domain of the present invention is connected to the N-terminus of the third domain through or without a linker peptide.

In a specific embodiment, the heavy chain of the second domain of the present invention is connected to the C-terminus of the third domain via or without a linker peptide.

Alternatively, in some embodiments, the second domain of the present invention comprises a light chain, and the third domain of the present invention is connected to the light chain of the second domain with or without a linker peptide.

In a specific embodiment, the light chain of the second domain of the present invention is connected to the N-terminus of the third domain through or without a linker peptide.

In a specific embodiment, the light chain of the second domain of the present invention is connected to the C-terminus of the third domain through or without a linker peptide.

In some embodiments, the CD80 mutant fusion polypeptide complex comprising the third domain of the present invention comprises a first polypeptide and a second polypeptide, and the second polypeptide may be present or absent.

In some embodiments, the first polypeptide includes the heavy chain of the second domain.

In one embodiment, the first polypeptide is is formed by covalently linking in sequence from the C-terminus to the N-terminus a CD80 IgV mutant polypeptide or a CD80 ECD mutant polypeptide, a linker peptide, a heavy chain of an antibody or an antigen-binding fragment thereof, a linker peptide, and a third domain, wherein the linker peptides may be the same or different and may exist or not exist independently of each other.

In one embodiment, the first polypeptide is formed by covalently linking in sequence from the C-terminus to the N-terminus a CD80 IgV mutant polypeptide or a CD80 ECD mutant polypeptide, a linker peptide, a heavy chain of an antibody or an antigen-binding fragment thereof, a linker peptide, and a third domain, wherein the linker peptides may be the same or different and may exist or not exist independently of each other.

Alternatively, in some embodiments, the second polypeptide includes a light chain of the second domain.

In some embodiments, the third domain is linked to the CD80 IgV mutant polypeptide or the CD80 ECD mutant polypeptide with or without a linker peptide.

In one embodiment, the third domain includes LAG-3 or an active fragment thereof, and the second domain specifically targets PD-1 or PD-L1.

In one embodiment, the first polypeptide comprises a sequence selected from the group consisting of: SEQ ID NO: 293, SEQ ID NO: 295; The second polypeptide comprises a sequence selected from the group consisting of: SEQ ID NO: 141.

In some embodiments, the linker peptide is absent.
In some embodiments, the linker peptide comprises one or more selected from GGGGS (SEQ ID NO: 92), GGGGSGGGGS (SEQ ID NO: 93), GGGGSGGGGSGGGGS (SEQ ID NO: 94), GGGGSGGGGSGGGGSGGGGS (SEQ ID NO: 95) and GGGGSGGGS (SEQ ID NO: 96).

Alternatively, in some embodiments, the CD80 mutant polypeptide can be combined with other forms of polypeptides to form the polypeptide complex through the above method.

In a fourth aspect, the present invention provides an immunoconjugate comprising the CD80 IgV mutant polypeptide as described in the first aspect, the CD80 ECD mutant polypeptide as described in the second aspect, or the CD80 mutant fusion polypeptide complex as described in the third aspect.

In a fifth aspect, the present invention provides a nucleotide molecule encoding the CD80 IgV mutant polypeptide as described in the first aspect, the CD80 ECD mutant polypeptide as described in the second aspect, or the CD80 mutant fusion polypeptide complex as described in the third aspect.

In a sixth aspect, the present invention provides a vector comprising the nucleotide molecule described in the fifth aspect.

In the seventh aspect, the present invention provides a host cell, comprising the CD80 IgV mutant polypeptide as described in the first aspect, the CD80 ECD mutant polypeptide as described in the second aspect, the CD80 mutant fusion polypeptide complex as described in the third aspect, the nucleotide molecule as described in the fifth aspect and/or the vector as described in the sixth aspect.

In an eighth aspect, the present invention provides a composition comprising the CD80 IgV mutant polypeptide as described in the first aspect, the CD80 ECD mutant polypeptide as described in the second aspect, the CD80 mutant fusion polypeptide complex as described in the third aspect, the immunoconjugate as described in the fourth aspect, the nucleotide molecule as described in the fifth aspect or the vector as described in the sixth aspect and a pharmaceutically acceptable carrier.

In the ninth aspect, the present invention provides a method for preparing the CD80 IgV mutant polypeptide as described in the first aspect, the CD80 ECD mutant polypeptide as described in the second aspect, or the CD80 mutant fusion polypeptide complex as described in the third aspect, comprising culturing the host cell as described in the seventh aspect to express the CD80 IgV mutant polypeptide, CD80 ECD mutant polypeptide and/or CD80 mutant fusion polypeptide complex.

In some embodiments, the present invention provides a method for expressing the polypeptide and polypeptide complex, which comprises using the host cell described in the present invention to express the CD80 mutant polypeptide or its fusion polypeptide complex described in the present invention.

In some embodiments, the present invention provides a method for producing the polypeptide and polypeptide complex, the method comprising introducing the polynucleotide encoding the antibody polypeptide complex of the present invention into a host cell, expressing the first polypeptide, with or without the second polypeptide, wherein the first polypeptide and the second polypeptide, or the first polypeptide itself can form a stable dimer, and the stable dimer can be maintained by a natural bond or/and a stable dimer comprising at least one non-natural interchain bond, the first polypeptide and the second polypeptide are assembled into a stable polymer in the host cell, and the stable complex is formed.

In certain embodiments, the present invention also provides a method for isolating and purifying the CD80 mutant polypeptide or its fusion polypeptide complex.

In the tenth aspect, the present invention provides a method for regulating T cell activity, comprising contacting the CD80 IgV mutant polypeptide as described in the first aspect, the CD80 ECD mutant polypeptide as described in the second aspect, or the CD80 mutant fusion polypeptide complex as described in the third aspect and/or the composition as described in the eighth aspect with T cells to stimulate their activation. The method is not limited to the protocol used in the examples, and can be extended to other protocols that have been published or developed in the future and can be used to evaluate T cell activation.

In the eleventh aspect, the present invention provides a method for inhibiting the growth or proliferation of tumor cells, comprising administering the CD80 IgV mutant polypeptide as described in the first aspect, the CD80 ECD mutant polypeptide as described in the second aspect, the CD80 mutant fusion polypeptide complex as described in the third aspect, the immunoconjugate as described in the fourth aspect, the nucleotide molecule as described in the fifth aspect, the vector as described in the sixth aspect, the host cell as described in the seventh aspect and/or the composition as described in the eighth aspect.

In the twelfth aspect, the present invention provides the use of the CD80 IgV mutant polypeptide as described in the first aspect, the CD80 ECD mutant polypeptide as described in the second aspect, the CD80 mutant fusion polypeptide complex as described in the third aspect, the immunoconjugate as described in the fourth aspect, the nucleotide molecule as described in the fifth aspect, the vector as described in the sixth aspect, the host cell as described in the seventh aspect and/or the composition as described in the eighth aspect in the preparation of a medicament for preventing, improving and/or treating tumors or cancer.

The following provides exemplary first polypeptide amino acid sequence of a functional fusion polypeptide complexes where the CD80 IgV mutants and the N-terminus of the sugemalimab antibody heavy chain are covalently linked via a linker peptide: E35N/A71E (SEQ ID NO: 97), E35N/A71N (SEQ ID NO: 98), E35N/A71Q (SEQ ID NO: 99), E35N/A71T (SEQ ID NO: 100), E35S/A71E (SEQ ID NO: 101), E35S/A71N (SEQ ID NO: 102), E35S/A71Q (SEQ ID NO: 103), E35S/A71T (SEQ ID NO: 104), E35Q/A71E (SEQ ID NO: 105), E35Q/A71N (SEQ ID NO: 106), E35Q/A71Q (SEQ ID NO: 107), E35Q/A71T (SEQ ID NO: 108), E35T/A71E (SEQ ID NO: 109), E35T/A71N (SEQ ID NO: 110), E35T/A71Q (SEQ ID NO: 112), E35T/A71T (SEQ ID NO: 113), M47A/I69Y/A71E/V83L (SEQ ID NO: 114), M47A/I69W/A71E/V83L (SEQ ID NO: 115), M47A/I69Y/A71E (SEQ ID NO: 116), M47A/I69W/A71E (SEQ ID NO: 117), I69Y/A71E/V83L (SEQ ID NO: 118), I69W/A71E/V83L (SEQ ID NO: 119), I69Y/A71E (SEQ ID NO: 120), I69W/A71E (SEQ ID NO: 121), H18W/A26M/A71E/L85N (SEQ ID NO: 122), H18W/A26M/A71E/L85D (SEQ ID NO: 123), H18W/A26C/A71E/L85N (SEQ ID NO: 124), H18W/A26C/A71E/L85D (SEQ ID NO: 125), A26M/A71E/L85N (SEQ ID NO: 126), A26M/A71E/L85D (SEQ ID NO: 127), A26C/A71E/L85D (SEQ ID NO: 128), A26C/A71E/L85N (SEQ ID NO: 129), I69L/A71E/V83L (SEQ ID NO: 130), H18W/A26V/E35N/A71E/L85N (SEQ ID NO: 131), T13K/M42L/M47H/A71E (SEQ ID NO: 132), T13K/M42L/M47Q/A71E (SEQ ID NO: 133), T13K/M42L/A71E (SEQ ID NO: 134), T13K/M42T/M47H/A71E (SEQ ID NO: 135), T13K/M42T/M47T/A71E (SEQ ID NO: 136), T13K/M42T/A71E (SEQ ID NO: 136), M47H/I69Y/A71E/V83L (SEQ ID NO: 137), M47Q/I69Y/A71E/V83L (SEQ ID NO: 138), M47K/I69Y/A71E/V83L (SEQ ID NO: 139), and the first polypeptide amino acid sequence (SEQ ID NO: 140) of a functional fusion polypeptide complex where the wild-type CD80 IgV is covalently linked to the N-terminus of the sugemalimab antibody heavy chain via a linker peptide. The above first polypeptides are respectively assembled into the functional fusion polypeptide complex with the sugemalimab antibody light chain second polypeptide (SEQ ID NO: 141).

The following provides an exemplary first polypeptide amino acid sequence of a functional fusion polypeptide complexes where the CD80 ECD mutants and the N-terminus of the sugemalimab antibody heavy chain are covalently linked via a linker peptide: E35N/A71E (SEQ ID NO: 142), E35N/A71N (SEQ ID NO: 143), E35N/A71Q (SEQ ID NO: 144), E35N/A71T (SEQ ID NO: 145), E35S/A71E (SEQ ID NO: 146), E35S/A71N (SEQ ID NO: 147), E35S/A71Q (SEQ ID NO: 148), E35S/A71T (SEQ ID NO: 149), E35Q/A71E (SEQ ID NO: 150), E35Q/A71N (SEQ ID NO: 151), E35Q/A71Q (SEQ ID NO: 152), E35Q/A71T (SEQ ID NO: 153), E35T/A71E (SEQ ID NO: 154), E35T/A71N (SEQ ID NO: 155), E35T/A71Q (SEQ ID NO: 156), E35T/A71T (SEQ ID NO: 157). The above first polypeptides are respectively assembled into the functional fusion polypeptide complex with the second polypeptide of the light chain of sugemalimab antibody (SEQ ID NO: 141).

The following provides exemplary functional fusion polypeptide amino acid sequence where CD80 ECD mutants are respectively covalently linked to the N-terminus of the Fc domain of human immunoglobulin IgG4: E35N/A71E (SEQ ID NO: 158), E35N/A71N (SEQ ID NO: 159), E35N/A71Q (SEQ ID NO: 160), E35N/A71T (SEQ ID NO: 161), E35S/A71E (SEQ ID NO: 162), E35S/A71N (SEQ ID NO: 163), E35S/A71Q (SEQ ID NO: 164), E35S/A71T (SEQ ID NO: 165), E35Q/A71E (SEQ ID NO: 166), E35Q/A71N (SEQ ID NO: 167), E35Q/A71Q (SEQ ID NO: 168), E35Q/A71T (SEQ ID NO: 169), E35T/A71E (SEQ ID NO: 170), E35T/A71N (SEQ ID NO: 171), E35T/A71Q (SEQ ID NO: 172), E35T/A71T (SEQ ID NO: 173), and a functional fusion polypeptide amino acid sequence (SEQ ID NO: 174) where unmutated CD80 ECD and the N-terminus of the Fc domain of human immunoglobulin IgG4 are covalently linked.

The following provides exemplary functional fusion polypeptide amino acid sequence where CD80 IgV mutants are respectively covalently linked to the N-terminus of the Fc domain of human immunoglobulin IgG4: E35N/A71E (SEQ ID NO: 175), E35N/A71N (SEQ ID NO: 176), E35N/A71Q (SEQ ID NO: 177), E35N/A71T (SEQ ID NO: 178), E35S/A71E (SEQ ID NO: 179), E35S/A71N (SEQ ID NO: 180), E35S/A71Q (SEQ ID NO: 181), E35S/A71T (SEQ ID NO: 182), E35Q/A71E (SEQ ID NO: 183), E35Q/A71N (SEQ ID NO: 184), E35Q/A71Q (SEQ ID NO: 185), E35Q/A71T (SEQ ID NO: 186), E35T/A71E (SEQ ID NO: 187), E35T/A71N (SEQ ID NO: 188), E35T/A71Q (SEQ ID NO: 189), E35T/A71T (SEQ ID NO: 190), and a functional fusion polypeptide amino acid sequence (SEQ ID NO: 191) where the unmutated CD80 IgV and the N-terminus of the Fc domain of human immunoglobulin IgG4 are covalently linked.

The following provides exemplary functional fusion polypeptide complex first polypeptide amino acid sequence where CD80 ECD mutant and the anti-LAG3 antibody Relatlimab heavy chain N-terminus are covalently linked via a linker peptide: E35N/A71E (SEQ ID NO: 192), E35N/A71N (SEQ ID NO: 193), E35N/A71Q (SEQ ID NO: 194), E35N/A71T (SEQ ID NO: 195), and unmutated CD80 ECD (SEQ ID NO: 196). The above first polypeptide is respectively assembled into the functional fusion polypeptide complex with the second polypeptide of the light chain of Relatlimab antibody (SEQ ID NO: 197).

The following provides exemplary functional fusion polypeptide complex first polypeptide amino acid sequence where CD80 IgV mutant and the anti-LAG3 antibody Relatlimab heavy chain N-terminus are covalently linked via a linker peptide: E35N/A71E (SEQ ID NO: 198), E35N/A71N (SEQ ID NO: 199), E35N/A71Q (SEQ ID NO: 200), E35N/A71T (SEQ ID NO: 201), and unmutated CD80 IgV (SEQ ID NO: 202). The above first polypeptide is respectively assembled into the functional fusion polypeptide complex with the second polypeptide of the light chain of Relatlimab antibody (SEQ ID NO: 197).

The following provides exemplary functional fusion polypeptide complex first polypeptide amino acid sequence where CD80 ECD mutant and the anti-CD3 antibody CD3B219 heavy chain N-terminal are covalently linked through a linker peptide: E35N/A71E (SEQ ID NO: 203), E35N/A71N (SEQ ID NO: 204), E35N/A71Q (SEQ ID NO: 205), E35N/A71T (SEQ ID NO: 206), and unmutated CD80 ECD (SEQ ID NO: 207). The above first polypeptide is respectively assembled into the functional fusion polypeptide complex with the second polypeptide of CD3B219 antibody light chain (SEQ ID NO: 208).

The following provides an exemplary CD80 IgV mutant and the anti-CD3 antibody CD3B219 heavy chain N-terminal covalently linked via a linker peptide to form a functional fusion polypeptide complex first polypeptide amino acid sequence: E35N/A71E (SEQ ID NO: 209), E35N/A71N (SEQ ID NO: 210), E35N/A71Q (SEQ ID NO: 211), E35N/A71T (SEQ ID NO: 212), and unmutated CD80 IgV (SEQ ID NO: 213), the above first polypeptide is respectively combined with the second polypeptide of CD3B219 antibody light chain (SEQ ID NO: 208) are assembled into the functional fusion polypeptide complex.

In certain embodiments, the functional antibody Abs-353 is composed of a chimeric antibody Abs-353 first polypeptide (SEQ ID NO: 97) (formed by CD80 IgV mutant E35N/A71E via a linker peptide (SEQ ID NO: 95) covalently linked to the N-terminus of the sugemalimab antibody heavy chain) and sugemalimab light chain second polypeptide (SEQ ID NO: 141). Abs-353 functional antibody is assembled by introducing a polynucleotide sequence (SEQ ID NO: 214) vector expressing Abs-353 first polypeptide and a polynucleotide sequence (SEQ ID NO: 215) vector expressing Abs-353 second polypeptide into a host cell for expression.

In certain embodiments, the functional antibody Abs-354 is composed of a chimeric antibody Abs-354 first polypeptide (SEQ ID NO: 98) (formed by CD80 IgV mutant E35N/A71N via a linker peptide (SEQ ID NO: 95) covalently linked to the N-terminus of the sugemalimab antibody heavy chain) and sugemalimab light chain second polypeptide (SEQ ID NO: 141). **Abs-354** functional antibody is assembled by introducing a polynucleotide sequence (SEQ ID NO: 216) vector expressing the Abs-354 first polypeptide and a polynucleotide sequence (SEQ ID NO: 215) vector expressing the Abs-354 second polypeptide into a host cell for expression_{∘}

In certain embodiments, the functional antibody Abs-355 is composed of a chimeric antibody Abs-355 first polypeptide (SEQ ID NO: 99) (formed by CD80 IgV mutant E35N/A71Q via a linker peptide (SEQ ID NO: 95) covalently linked to the N-terminus of the sugemalimab antibody heavy chain) and sugemalimab light chain second polypeptide (SEQ ID NO: 141). Abs-355 functional antibody is assembled by introducing a polynucleotide sequence (SEQ ID NO: 217) vector expressing Abs-355 first polypeptide and a polynucleotide sequence (SEQ ID NO: 215) vector expressing Abs-355 second polypeptide into a host cell for expression.

In certain embodiments, the functional antibody Abs-356 is composed of a chimeric antibody Abs-356 first polypeptide (SEQ ID NO: 100) (formed by CD80 IgV mutant E35N/A71T via a linker peptide (SEQ ID NO: 95) covalently linked to the N-terminus of the sugemalimab antibody heavy chain) and sugemalimab light chain second polypeptide (SEQ ID NO: 141). **Abs-356** functional antibody is assembled by introducing a polynucleotide sequence (SEQ ID NO: 218) vector expressing Abs-356 first polypeptide and a polynucleotide sequence (SEQ ID NO: 215) vector expressing Abs-356 second polypeptide into a host cell for expression.

In certain embodiments, the functional antibody Abs-357 is composed of a chimeric antibody Abs-357 first polypeptide (SEQ ID NO: 101) (formed by CD80 IgV mutant E35S/A71E via a linker peptide (SEQ ID NO: 95) covalently linked to the N-terminus of the sugemalimab antibody heavy chain) and sugemalimab light chain second polypeptide (SEQ ID NO: 141). **Abs-357** functional antibody is assembled by introducing a polynucleotide sequence (SEQ ID NO: 219) vector expressing Abs-357 first polypeptide and a polynucleotide sequence (SEQ ID NO: 215) vector expressing Abs-357 second polypeptide into a host cell for expression.

In certain embodiments, the functional antibody Abs-358 is composed of (formed by CD80 IgV mutant E35S/A71N via a linker peptide (SEQ ID NO: 95) covalently linked to the N-terminus of the sugemalimab antibody heavy chain) a chimeric antibody Abs-358 first polypeptide (SEQ ID NO: 102) and sugemalimab light chain second polypeptide (SEQ ID NO: 141). **Abs-358** functional antibody is assembled by introducing a polynucleotide sequence (SEQ ID NO: 220) vector expressing Abs-358 first polypeptide and a polynucleotide sequence (SEQ ID NO: 215) vector expressing Abs-358 second polypeptide into a host cell for expression.

In certain embodiments, the functional antibody Abs-359 is composed of (formed by CD80 IgV mutant E35S/A71Q via a linker peptide (SEQ ID NO: 95) covalently linked to the N-terminus of the sugemalimab antibody heavy chain) a chimeric antibody Abs- first polypeptide (SEQ ID NO: 103) and sugemalimab light chain second polypeptide (SEQ ID NO: 141). **Abs-359** functional antibody is assembled by introducing a polynucleotide sequence (SEQ ID NO: 221) vector expressing Abs-359 first polypeptide and a polynucleotide sequence (SEQ ID NO: 215) vector expressing Abs-359 second polypeptide into a host cell for expression.

In certain embodiments, the functional antibody Abs-360 is composed of (formed by CD80 IgV mutant E35S/A71T via a linker peptide (SEQ ID NO: 95) covalently linked to the N-terminus of the sugemalimab antibody heavy chain) a chimeric antibody Abs- first polypeptide (SEQ ID NO: 104) and sugemalimab light chain second polypeptide (SEQ ID NO: 141). **Abs-360** functional antibody is assembled by introducing a polynucleotide sequence (SEQ ID NO: 222) vector expressing Abs-360 first polypeptide and a polynucleotide sequence (SEQ ID NO: 215) vector expressing Abs-360 second polypeptide into a host cell for expression.

In certain embodiments, the functional antibody Abs-361 is composed of (formed by CD80 IgV mutant E35Q/A71E via a linker peptide (SEQ ID NO: 95) covalently linked to the N-terminus of the sugemalimab antibody heavy chain) a chimeric antibody Abs-361 first polypeptide (SEQ ID NO: 105) and sugemalimab light chain second polypeptide (SEQ ID NO: 141). Abs-361 functional antibody is assembled by introducing a polynucleotide sequence (SEQ ID NO: 223) vector expressing Abs-361 first polypeptide and a polynucleotide sequence (SEQ ID NO: 215) vector expressing Abs-361 second polypeptide into a host cell for expression.

In certain embodiments, the functional antibody Abs-362 is composed of (formed by CD80 IgV mutant E35Q/A71N via a linker peptide (SEQ ID NO: 95) covalently linked to the N-terminus of the sugemalimab antibody heavy chain) a chimeric antibody Abs-362 first polypeptide (SEQ ID NO: 106) and sugemalimab light chain second polypeptide (SEQ ID NO: 141). Abs-362 functional antibody is assembled by introducing a polynucleotide sequence (SEQ ID NO: 224) vector expressing Abs-362 first polypeptide and a polynucleotide sequence (SEQ ID NO: 215) vector expressing Abs-362 second polypeptide into a host cell for expression.

In certain embodiments, the functional antibody Abs-363 is composed of a chimeric antibody Abs-363 first polypeptide (SEQ ID NO: 107) (formed by CD80 IgV mutant E35Q/A71Q via a linker peptide (SEQ ID NO: 95) covalently linked to the N-terminus of the sugemalimab antibody heavy chain) and sugemalimab light chain second polypeptide (SEQ ID NO: 141). Abs-363 functional antibody is assembled by introducing a polynucleotide sequence (SEQ ID NO: 225) vector expressing Abs-363 first polypeptide and a polynucleotide sequence (SEQ ID NO: 215) vector expressing Abs-363 second polypeptide into a host cell for expression.

In certain embodiments, the functional antibody Abs-364 is composed of a chimeric antibody Abs-364 first polypeptide (SEQ ID NO: 108) (formed by CD80 IgV mutant E35Q/A71T via a linker peptide (SEQ ID NO: 95) covalently linked to the N-terminus of the sugemalimab antibody heavy chain) and sugemalimab light chain second polypeptide (SEQ ID NO: 141). Abs-364 functional antibody is assembled by introducing a polynucleotide sequence (SEQ ID NO: 226) vector expressing Abs-364 first polypeptide and a polynucleotide sequence (SEQ ID NO: 215) vector expressing Abs-364 second polypeptide into a host cell for expression.

In certain embodiments, the functional antibody Abs-365 is composed of a chimeric antibody Abs-365 first polypeptide (SEQ ID NO: 109) (formed by CD80 IgV mutant E35T/A71E via a linker peptide (SEQ ID NO: 95) covalently linked to the N-terminus of the sugemalimab antibody heavy chain) and sugemalimab light chain second polypeptide (SEQ ID NO: 141). Abs-365 functional antibody is assembled by introducing a polynucleotide sequence (SEQ ID NO: 227) vector expressing Abs-365 first polypeptide and a polynucleotide sequence (SEQ ID NO: 215) vector expressing Abs-365 second polypeptide into a host cell for expression.

In certain embodiments, the functional antibody Abs-366 is composed of a chimeric antibody Abs-366 first polypeptide (SEQ ID NO: 110) (formed by CD80 IgV mutant E35T/A71N via a linker peptide (SEQ ID NO: 95) covalently linked to the N-terminus of the sugemalimab antibody heavy chain) and sugemalimab light chain second polypeptide (SEQ ID NO: 141). Abs-366 functional antibody is assembled by introducing a polynucleotide sequence (SEQ ID NO: 228) vector expressing Abs-366 first polypeptide and a polynucleotide sequence (SEQ ID NO: 215) vector expressing Abs-366 second polypeptide into a host cell for expression.

In certain embodiments, the functional antibody Abs-367 is composed of a chimeric antibody Abs-367 first polypeptide (SEQ ID NO: 111) (formed by CD80 IgV mutant E35T/A71Q via a linker peptide (SEQ ID NO: 95) covalently linked to the N-terminus of the sugemalimab antibody heavy chain) and sugemalimab light chain second polypeptide (SEQ ID NO: 141). Abs-367 functional antibody is assembled by introducing a polynucleotide sequence (SEQ ID NO: 229) vector expressing Abs-367 first polypeptide and a polynucleotide sequence (SEQ ID NO: 215) vector expressing Abs-367 second polypeptide into a host cell for expression.

In certain embodiments, the functional antibody Abs-368 is composed of (formed by CD80 IgV mutant E35T/A71T via a linker peptide (SEQ ID NO: 95) covalently linked to the N-terminus of the sugemalimab antibody heavy chain) a chimeric antibody Abs-368 first polypeptide (SEQ ID NO: 112) and sugemalimab light chain second polypeptide (SEQ ID NO: 141). Abs-368 functional antibody is assembled by introducing a polynucleotide sequence (SEQ ID NO: 230) vector expressing Abs-368 first polypeptide and a polynucleotide sequence (SEQ ID NO: 215) vector expressing Abs-368 second polypeptide into a host cell for expression.

In certain embodiments, the functional antibody Abs-247 is composed of a chimeric antibody Abs-247 first polypeptide (SEQ ID NO: 113) (formed by CD80 IgV mutant M47A/I69Y/A71E/V83L via a linker peptide (SEQ ID NO: 95) covalently linked to the N-terminus of the sugemalimab antibody heavy chain) and sugemalimab light chain second polypeptide (SEQ ID NO: 141). Abs-247 functional antibody is assembled by introducing a polynucleotide sequence (SEQ ID NO: 231) vector expressing Abs-247 first polypeptide and a polynucleotide sequence (SEQ ID NO: 215) vector expressing Abs-247 second polypeptide into a host cell for expression.

In certain embodiments, the functional antibody Abs-248 is composed of a chimeric antibody Abs-248 first polypeptide (SEQ ID NO: 114) (formed by CD80 IgV mutant M47A/I69W/A71E/V83L via a linker peptide (SEQ ID NO: 95) covalently linked to the N-terminus of the sugemalimab antibody heavy chain) and sugemalimab light chain second polypeptide (SEQ ID NO: 141). Abs-248 functional antibody is assembled by introducing a polynucleotide sequence (SEQ ID NO: 232) vector expressing Abs-248 first polypeptide and a polynucleotide sequence (SEQ ID NO: 215) vector expressing Abs-248 second polypeptide into a host cell for expression.

In certain embodiments, the functional antibody Abs-249 is composed of a chimeric antibody Abs-249 first polypeptide (SEQ ID NO: 115) (formed by CD80 IgV mutant M47A/I69Y/A71E via a linker peptide (SEQ ID NO: 95) covalently linked to the N-terminus of the sugemalimab antibody heavy chain) and sugemalimab light chain second polypeptide (SEQ ID NO: 141). Abs-249 functional antibody is assembled by introducing a polynucleotide sequence (SEQ ID NO: 233) vector expressing Abs-249 first polypeptide and a polynucleotide sequence (SEQ ID NO: 215) vector expressing Abs-249 second polypeptide into a host cell for expression.

In certain embodiments, the functional antibody Abs-250 is composed of a chimeric antibody Abs-250 first polypeptide (SEQ ID NO: 116) (formed by CD80 IgV mutant M47A/I69W/A71E via a linker peptide (SEQ ID NO: 95) covalently linked to the N-terminus of the sugemalimab antibody heavy chain) and sugemalimab light chain second polypeptide (SEQ ID NO: 141). Abs-250 functional antibody is assembled by introducing a polynucleotide sequence (SEQ ID NO: 234) vector expressing Abs-250 first polypeptide and a polynucleotide sequence (SEQ ID NO: 215) vector expressing Abs-250 second polypeptide into a host cell for expression.

In certain embodiments, the functional antibody Abs-251 is composed of (formed by CD80 IgV mutant I69Y/A71E/V83L via a linker peptide (SEQ ID NO: 95) covalently linked to the N-terminus of the sugemalimab antibody heavy chain) a chimeric antibody Abs-251 first polypeptide (SEQ ID NO: 117) and sugemalimab light chain second polypeptide (SEQ ID NO: 141). Abs-251 functional antibody is assembled by introducing a polynucleotide sequence (SEQ ID NO: 235) vector expressing Abs-251 first polypeptide and a polynucleotide sequence (SEQ ID NO: 215) vector expressing Abs-251 second polypeptide into a host cell for expression.

In certain embodiments, the functional antibody Abs-252 is composed of a chimeric antibody Abs-252 first polypeptide (SEQ ID NO: 118) (formed by CD80 IgV mutant I69W/A71E/V83L via a linker peptide (SEQ ID NO: 95) covalently linked to the N-terminus of the sugemalimab antibody heavy chain) and sugemalimab light chain second polypeptide (SEQ ID NO: 141). Abs-252 functional antibody is assembled by introducing a polynucleotide sequence (SEQ ID NO: 236) vector expressing Abs-252 first polypeptide and a polynucleotide sequence (SEQ ID NO: 215) vector expressing Abs-252 second polypeptide into a host cell for expression

In certain embodiments, the functional antibody Abs-253 is composed of a chimeric antibody Abs-253 first polypeptide (SEQ ID NO: 119) (formed by CD80 IgV mutant I69Y/A71E via a linker peptide (SEQ ID NO: 95) covalently linked to the N-terminus of the sugemalimab antibody heavy chain) and sugemalimab light chain second polypeptide (SEQ ID NO: 141). Abs-253 functional antibody is assembled by introducing a polynucleotide sequence (SEQ ID NO: 237) vector expressing Abs-253 first polypeptide and a polynucleotide sequence (SEQ ID NO: 215) vector expressing Abs-253 second polypeptide into a host cell for expression.

In certain embodiments, the functional antibody Abs-254 is composed of a chimeric antibody Abs-254 first polypeptide (SEQ ID NO: 120) (formed by CD80 IgV mutant I69W/A71E via a linker peptide (SEQ ID NO: 95) covalently linked to the N-terminus of the sugemalimab antibody heavy chain) and sugemalimab light chain second polypeptide (SEQ ID NO: 141). Abs-254 functional antibody is assembled by introducing a polynucleotide sequence (SEQ ID NO: 238) vector expressing Abs-254 first polypeptide and a polynucleotide sequence (SEQ ID NO: 215) vector expressing Abs-254 second polypeptide into a host cell for expression.

In certain embodiments, the functional antibody Abs-255 is composed of a chimeric antibody Abs-255 first polypeptide (SEQ ID NO: 121) (formed by CD80 IgV mutant H18W/A26M/A71E/L85N via a linker peptide (SEQ ID NO: 95) covalently linked to the N-terminus of the sugemalimab antibody heavy chain) and sugemalimab light chain second polypeptide (SEQ ID NO: 141). Abs-255 functional antibody is assembled by introducing a polynucleotide sequence (SEQ ID NO: 239) vector expressing Abs-255 first polypeptide and a polynucleotide sequence (SEQ ID NO: 215) vector expressing Abs-255 second polypeptide into a host cell for expression.

In certain embodiments, the functional antibody Abs-256 is composed of a chimeric antibody Abs-256 first polypeptide (SEQ ID NO: 122) (formed by CD80 IgV mutant H18W/A26M/A71E/L85D via a linker peptide (SEQ ID NO: 95) covalently linked to the N-terminus of the sugemalimab antibody heavy chain) and sugemalimab light chain second polypeptide (SEQ ID NO: 141). Abs-256 functional antibody is assembled by introducing a polynucleotide sequence (SEQ ID NO: 240) vector expressing Abs-256 first polypeptide and a polynucleotide sequence (SEQ ID NO: 215) vector expressing Abs-256 second polypeptide into a host cell for expression.

In certain embodiments, the functional antibody Abs-257 is composed of a chimeric antibody Abs-257 first polypeptide (SEQ ID NO: 123) (formed by CD80 IgV mutant H18W/A26C/A71E/L85N via a linker peptide (SEQ ID NO: 95) covalently linked to the N-terminus of the sugemalimab antibody heavy chain) and sugemalimab light chain second polypeptide (SEQ ID NO: 141). Abs-257 functional antibody is assembled by introducing a polynucleotide sequence (SEQ ID NO: 241) vector expressing Abs-257 first polypeptide and a polynucleotide sequence (SEQ ID NO: 215) vector expressing Abs-257 second polypeptide into a host cell for expression.

In certain embodiments, the functional antibody Abs-258 is composed of a chimeric antibody Abs-258 first polypeptide (SEQ ID NO: 124) (formed by CD80 IgV mutant H18W/A26C/A71E/L85D via a linker peptide (SEQ ID NO: 95) covalently linked to the N-terminus of the sugemalimab antibody heavy chain) and sugemalimab light chain second polypeptide (SEQ ID NO: 141). Abs-258 functional antibody is assembled by introducing a polynucleotide sequence (SEQ ID NO: 242) vector expressing Abs-258 first polypeptide and a polynucleotide sequence (SEQ ID NO: 215) vector expressing Abs-258 second polypeptide into a host cell for expression.

In certain embodiments, the functional antibody Abs-259 is composed of a chimeric antibody Abs-259 first polypeptide (SEQ ID NO: 125) (formed by CD80 IgV mutant A26M/A71E/L85N via a linker peptide (SEQ ID NO: 95) covalently linked to the N-terminus of the sugemalimab antibody heavy chain) and sugemalimab light chain second polypeptide (SEQ ID NO: 141). Abs-259 functional antibody is assembled by introducing a polynucleotide sequence (SEQ ID NO: 243) vector expressing Abs-259 first polypeptide and a polynucleotide sequence (SEQ ID NO: 215) vector expressing Abs-259 second polypeptide into a host cell for expression.

In certain embodiments, the functional antibody Abs-260 is composed of a chimeric antibody Abs-260 first polypeptide (SEQ ID NO: 126) (formed by CD80 IgV mutant A26M/A71E/L85D via a linker peptide (SEQ ID NO: 95) covalently linked to the N-terminus of the sugemalimab antibody heavy chain) and sugemalimab light chain second polypeptide (SEQ ID NO: 141). Abs-260 functional antibody is assembled by introducing a polynucleotide sequence (SEQ ID NO: 244) vector expressing Abs-260 first polypeptide and a polynucleotide sequence (SEQ ID NO: 215) vector expressing Abs-260 second polypeptide into a host cell for expression.

In certain embodiments, the functional antibody Abs-261 is composed of a chimeric antibody Abs-261 first polypeptide (SEQ ID NO: 127) (formed by CD80 IgV mutant A26C/A71E/L85D via a linker peptide (SEQ ID NO: 95) covalently linked to the N-terminus of the sugemalimab antibody heavy chain) and sugemalimab light chain second polypeptide (SEQ ID NO: 141). Abs-261 functional antibody is assembled by introducing a polynucleotide sequence (SEQ ID NO: 245) vector expressing Abs-261 first polypeptide and a polynucleotide sequence (SEQ ID NO: 215) vector expressing Abs-261 second polypeptide into a host cell for expression.

In certain embodiments, the functional antibody Abs-262 is composed of a chimeric antibody Abs-262 first polypeptide (SEQ ID NO: 128) (formed by CD80 IgV mutant A26C/A71E/L85N via a linker peptide (SEQ ID NO: 95) covalently linked to the N-terminus of the sugemalimab antibody heavy chain) and sugemalimab light chain second polypeptide (SEQ ID NO: 141). Abs-262 functional antibody is assembled by introducing a polynucleotide sequence (SEQ ID NO: 246) vector expressing Abs-262 first polypeptide and a polynucleotide sequence (SEQ ID NO: 215) vector expressing Abs-262 second polypeptide into a host cell for expression.

In certain embodiments, the functional antibody Abs-263 is composed of a chimeric antibody Abs-263 first polypeptide (SEQ ID NO: 129) (formed by CD80 IgV mutant I69L/A71E/V83L via a linker peptide (SEQ ID NO: 95) covalently linked to the N-terminus of the sugemalimab antibody heavy chain) and sugemalimab light chain second polypeptide (SEQ ID NO: 141). Abs-263 functional antibody is assembled by introducing a polynucleotide sequence (SEQ ID NO: 247) vector expressing Abs-263 first polypeptide and a polynucleotide sequence (SEQ ID NO: 215) vector expressing Abs-263 second polypeptide into a host cell for expression.

In certain embodiments, the functional antibody Abs-264 is composed of a chimeric antibody Abs-264 first polypeptide (SEQ ID NO: 130) (formed by CD80 IgV mutant H18W/A26V/E35N/A71E/L85N via a linker peptide (SEQ ID NO: 95) covalently linked to the N-terminus of the sugemalimab antibody heavy chain) and sugemalimab light chain second polypeptide (SEQ ID NO: 141). Abs-264 functional antibody is assembled by introducing a polynucleotide sequence (SEQ ID NO: 248) vector expressing Abs-264 first polypeptide and a polynucleotide sequence (SEQ ID NO: 215) vector expressing Abs-264 second polypeptide into a host cell for expression.

In certain embodiments, the functional antibody Abs-265 is composed of a chimeric antibody Abs-265 first polypeptide (SEQ ID NO: 131) (formed by CD80 IgV mutant T13K/M42L/M47H/A71E via a linker peptide (SEQ ID NO: 95) covalently linked to the N-terminus of the sugemalimab antibody heavy chain) and sugemalimab light chain second polypeptide (SEQ ID NO: 141). Abs-265 functional antibody is assembled by introducing a polynucleotide sequence (SEQ ID NO: 249) vector expressing Abs-265 first polypeptide and a polynucleotide sequence (SEQ ID NO: 215) vector expressing Abs-265 second polypeptide into a host cell for expression.

In certain embodiments, the functional antibody Abs-266 is composed of a chimeric antibody Abs-266 first polypeptide (SEQ ID NO: 132) (formed by CD80 IgV mutant T13K/M42L/M47Q/A71E via a linker peptide (SEQ ID NO: 95) covalently linked to the N-terminus of the sugemalimab antibody heavy chain) and sugemalimab light chain second polypeptide (SEQ ID NO: 141). Abs-266 functional antibody is assembled by introducing a polynucleotide sequence (SEQ ID NO: 250) vector expressing Abs-268 first polypeptide and a polynucleotide sequence (SEQ ID NO: 215) vector expressing Abs-266 second polypeptide into a host cell for expression.

In certain embodiments, the functional antibody Abs-267 is composed of a chimeric antibody Abs-267 first polypeptide (SEQ ID NO: 133) (formed by CD80 IgV mutant T13K/M42L/A71E via a linker peptide (SEQ ID NO: 95) covalently linked to the N-terminus of the sugemalimab antibody heavy chain) and sugemalimab light chain second polypeptide (SEQ ID NO: 141). Abs-267 functional antibody is assembled by introducing a polynucleotide sequence (SEQ ID NO: 251) vector expressing Abs-267 first polypeptide and a polynucleotide sequence (SEQ ID NO: 215) vector expressing Abs-267 second polypeptide into a host cell for expression.

In certain embodiments, the functional antibody Abs-268 is composed of a chimeric antibody Abs-268 first polypeptide (SEQ ID NO: 134) (formed by CD80 IgV mutant T13K/M42T/M47H/A71E via a linker peptide (SEQ ID NO: 95) covalently linked to the N-terminus of the sugemalimab antibody heavy chain) and sugemalimab light chain second polypeptide (SEQ ID NO: 141). Abs-268 functional antibody is assembled by introducing a polynucleotide sequence (SEQ ID NO: 252) vector expressing Abs-268 first polypeptide and a polynucleotide sequence (SEQ ID NO: 215) vector expressing Abs-268 second polypeptide into a host cell for expression.

In certain embodiments, the functional antibody Abs-269 is composed of a chimeric antibody Abs-269 first polypeptide (SEQ ID NO: 135) (formed by CD80 IgV mutant T13K/M42T/M47T/A71E via a linker peptide (SEQ ID NO: 95) covalently linked to the N-terminus of the sugemalimab antibody heavy chain) and sugemalimab light chain second polypeptide (SEQ ID NO: 141). Abs-269 functional antibody is assembled by introducing a polynucleotide sequence (SEQ ID NO: 253) vector expressing Abs- first polypeptide and a polynucleotide sequence (SEQ ID NO: 215) vector expressing Abs-269 second polypeptide into a host cell for expression.

In certain embodiments, the functional antibody Abs-270 is composed of a chimeric antibody Abs-270 first polypeptide (SEQ ID NO: 136) (formed by CD80 IgV mutant T13K/M42T/A71E via a linker peptide (SEQ ID NO: 95) covalently linked to the N-terminus of the sugemalimab antibody heavy chain) and sugemalimab light chain second polypeptide (SEQ ID NO: 141). Abs-270 functional antibody is assembled by introducing a polynucleotide sequence (SEQ ID NO: 254) vector expressing Abs-270 first polypeptide and a polynucleotide sequence (SEQ ID NO: 215) vector expressing Abs-270 second polypeptide into a host cell for expression.

In certain embodiments, the functional antibody Abs-271 is composed of a chimeric antibody Abs-271 first polypeptide (SEQ ID NO: 137) (formed by CD80 IgV mutant M47H/I69Y/A71E/V83L via a linker peptide (SEQ ID NO: 95) covalently linked to the N-terminus of the sugemalimab antibody heavy chain) and sugemalimab light chain second polypeptide (SEQ ID NO: 141). Abs-271 functional antibody is assembled by introducing a polynucleotide sequence (SEQ ID NO: 255) vector expressing Abs-271 first polypeptide and a polynucleotide sequence (SEQ ID NO: 215) vector expressing Abs-271 second polypeptide into a host cell for expression.

In certain embodiments, the functional antibody Abs-272 is composed of a chimeric antibody Abs-272 first polypeptide (SEQ ID NO: 138) (formed by CD80 IgV mutant M47Q/I69Y/A71E/V83L via a linker peptide (SEQ ID NO: 95) covalently linked to the N-terminus of the sugemalimab antibody heavy chain) and sugemalimab light chain second polypeptide (SEQ ID NO: 141). Abs-272 functional antibody is assembled by introducing a polynucleotide sequence (SEQ ID NO: 256) vector expressing Abs-272 first polypeptide and a polynucleotide sequence (SEQ ID NO: 215) vector expressing Abs-272 second polypeptide into a host cell for expression.

In certain embodiments, the functional antibody Abs-273 is composed of a chimeric antibody Abs-273 first polypeptide (SEQ ID NO: 139) (formed by CD80 IgV mutant M47K/I69Y/A71E/V83L via a linker peptide (SEQ ID NO: 95) covalently linked to the N-terminus of the sugemalimab antibody heavy chain) and sugemalimab light chain second polypeptide (SEQ ID NO: 141). Abs-273 functional antibody is assembled by introducing a polynucleotide sequence (SEQ ID NO: 257) vector expressing Abs-273 first polypeptide and a polynucleotide sequence (SEQ ID NO: 215) vector expressing Abs-273 second polypeptide into a host cell for expression.

In certain embodiments, the functional antibody Abs-99 is composed of a chimeric antibody Abs-99 first polypeptide (SEQ ID NO: 140) (formed by wild-type CD80 IgV via a linker peptide (SEQ ID NO: 95) covalently linked to the N-terminus of the sugemalimab antibody heavy chain) and sugemalimab light chain second polypeptide (SEQ ID NO: 141). Abs-99 functional antibody is assembled by introducing a polynucleotide sequence (SEQ ID NO: 258) vector expressing Abs-99 first polypeptide and a polynucleotide sequence (SEQ ID NO: 215) vector expressing Abs-99 second polypeptide into a host cell for expression.

In certain embodiments, the functional antibody Abs-509 is composed of a chimeric antibody Abs-509 first polypeptide (SEQ ID NO: 142) (formed by CD80 ECD mutant E35N/A71E via a linker peptide (SEQ ID NO: 95) covalently linked to the N-terminus of the sugemalimab antibody heavy chain) and sugemalimab light chain second polypeptide (SEQ ID NO: 141). Abs-509 functional antibody is assembled by introducing a polynucleotide sequence (SEQ ID NO: 259) vector expressing Abs-509 first polypeptide and a polynucleotide sequence (SEQ ID NO: 215) vector expressing Abs-509 second polypeptide into a host cell for expression.

In certain embodiments, the functional antibody Abs-510 is composed of a chimeric antibody Abs-510 first polypeptide (SEQ ID NO: 143) (formed by CD80 ECD mutant E35N/A71N via a linker peptide (SEQ ID NO: 95) covalently linked to the N-terminus of the sugemalimab antibody heavy chain) and sugemalimab light chain second polypeptide (SEQ ID NO: 141). Abs-510 functional antibody is assembled by introducing a polynucleotide sequence (SEQ ID NO: 260) vector expressing Abs-510 first polypeptide and a polynucleotide sequence (SEQ ID NO: 215) vector expressing Abs-510 second polypeptide into a host cell for expression.

In certain embodiments, the functional antibody Abs-511 is composed of a chimeric antibody Abs-511 first polypeptide (SEQ ID NO: 144) (formed by CD80 ECD mutant E35N/A71Q via a linker peptide (SEQ ID NO: 95) covalently linked to the N-terminus of the sugemalimab antibody heavy chain) and sugemalimab light chain second polypeptide (SEQ ID NO: 141). Abs-511 functional antibody is assembled by introducing a polynucleotide sequence (SEQ ID NO: 261) vector expressing Abs-511 first polypeptide and a polynucleotide sequence (SEQ ID NO: 215) vector expressing Abs-511 second polypeptide into a host cell for expression.

In certain embodiments, the functional antibody Abs-512 is composed of a chimeric antibody Abs-512 first polypeptide (SEQ ID NO: 145) (formed by CD80 ECD mutant E35N/A71T via a linker peptide (SEQ ID NO: 95) covalently linked to the N-terminus of the sugemalimab antibody heavy chain) and sugemalimab light chain second polypeptide (SEQ ID NO: 141). Abs-512 functional antibody is assembled by introducing a polynucleotide sequence (SEQ ID NO: 262) vector expressing Abs-512 first polypeptide and a polynucleotide sequence (SEQ ID NO: 215) vector expressing Abs-512 second polypeptide into a host cell for expression.

In certain embodiments, the functional antibody Abs-98 is composed of a chimeric antibody Abs-98 first polypeptide (SEQ ID NO: 263) (formed by wild-type CD80 ECD via a linker peptide (SEQ ID NO: 95) covalently linked to the N-terminus of the sugemalimab antibody heavy chain) and sugemalimab light chain second polypeptide (SEQ ID NO: 141). Abs-98 functional antibody is assembled by introducing a polynucleotide sequence (SEQ ID NO: 264) vector expressing Abs-98 first polypeptide and a polynucleotide sequence (SEQ ID NO: 215) vector expressing Abs-98 second polypeptide into a host cell for expression.

In certain embodiments, the functional antibody Abs-480 is composed of chimeric polypeptide Abs-480(SEQ ID NO: 158) (formed by CD80 ECD mutant E35N/A71E covalently linked to IgG4 Fc N-terminus). Abs-480 functional polypeptide complex is assembled by introducing a polynucleotide sequence (SEQ ID NO: 265) vector expressing Abs-480 polypeptide into a host cell for expression.

In certain embodiments, the functional antibody Abs-481 is composed of chimeric polypeptide Abs-481(SEQ ID NO: 159) (formed by CD80 ECD mutant E35N/A71N covalently linked to IgG4 Fc N-terminus). Abs-481 functional polypeptide complex is assembled by introducing a polynucleotide sequence (SEQ ID NO: 266) vector expressing Abs-481 polypeptide into a host cell for expression.

In certain embodiments, the functional antibody Abs-482 is composed of chimeric polypeptide Abs-482(SEQ ID NO: 160) (formed by CD80 ECD mutant E35N/A71Q covalently linked to IgG4 Fc N-terminus). Abs-482 functional polypeptide complex is assembled by introducing a polynucleotide sequence (SEQ ID NO: 267) vector expressing Abs-482 polypeptide into a host cell for expression.

In certain embodiments, the functional antibody Abs-484 is composed of chimeric polypeptide Abs-484(SEQ ID NO: 161) (formed by CD80 ECD mutant E35N/A71T covalently linked to IgG4 Fc N-terminus). Abs-484 functional polypeptide complex is assembled by introducing a polynucleotide sequence (SEQ ID NO: 268) vector expressing Abs-484 polypeptide into a host cell for expression.

In certain embodiments, the functional antibody Abs-100 is composed of chimeric polypeptide Abs-100(SEQ ID NO: 174) (formed by wild-type CD80 ECD covalently linked to IgG4 Fc N-terminus). Abs-100 functional polypeptide complex is assembled by introducing a polynucleotide sequence (SEQ ID NO: 269) vector expressing Abs-100 polypeptide into a host cell for expression.

In certain embodiments, the functional antibody Abs-427 is composed of chimeric polypeptide Abs-427(SEQ ID NO: 175) (formed by CD80 IgV mutant E35N/A71E covalently linked to IgG4 Fc N-terminus). Abs-427 functional polypeptide complex is assembled by introducing a polynucleotide sequence (SEQ ID NO: 270) vector expressing Abs-427 polypeptide into a host cell for expression.

In certain embodiments, the functional antibody Abs-428 is composed of chimeric polypeptide Abs-428 (SEQ ID NO: 176) (formed by CD80 IgV mutant E35N/A71N covalently linked to IgG4 Fc N-terminus). Abs-428 functional polypeptide complex is assembled by introducing a polynucleotide sequence (SEQ ID NO: 271) vector expressing Abs-428 polypeptide into a host cell for expression.

In certain embodiments, the functional antibody Abs-429 is composed of chimeric polypeptide Abs-429 (SEQ ID NO: 177) (formed by CD80 IgV mutant E35N/A71Q covalently linked toIgG4 Fc N-terminus). Abs-429 functional polypeptide complex is assembled by introducing a polynucleotide sequence (SEQ ID NO: 272) vector expressing Abs-429 polypeptide into a host cell for expression.

In certain embodiments, the functional antibody Abs-483 is composed of chimeric polypeptide Abs-483 (SEQ ID NO: 178) (formed by CD80 IgV mutant E35N/A71T covalently linked toIgG4 Fc N-terminus). Abs-483 functional polypeptide complex is assembled by introducing a polynucleotide sequence (SEQ ID NO: 273) vector expressing Abs-483 polypeptide into a host cell for expression.

In certain embodiments, the functional antibody Abs-504 is composed of chimeric polypeptide Abs-504(SEQ ID NO: 191) (formed by wild-type CD80 IgV covalently linked to IgG4 Fc N-terminus). Abs-504 functional polypeptide complex is assembled by introducing a polynucleotide sequence (SEQ ID NO: 274) vector expressing Abs-504 polypeptide into a host cell for expression.

In certain embodiments, the functional antibody Abs-505 is composed of a chimeric antibody Abs-505 first polypeptide (SEQ ID NO: 198) (formed by CD80 IgV mutant E35N/A71E via a linker peptide (SEQ ID NO: 95) covalently linked to Relatlimab antibody heavy chain N-terminus) and Relatlimab light chain second polypeptide (SEQ ID NO: 197). Abs-505 functional antibody is assembled by introducing a polynucleotide sequence (SEQ ID NO: 276) vector expressing Abs-505 first polypeptide and a polynucleotide sequence (SEQ ID NO: 275) vector expressing Abs-505 second polypeptide into a host cell for expression.

In certain embodiments, the functional antibody Abs-513 is composed of (formed by CD80 IgV mutant E35N/A71N via a linker peptide (SEQ ID NO: 95) covalently linked to Relatlimab antibody heavy chain N-terminus) a chimeric antibody Abs-513 first polypeptide (SEQ ID NO: 199) and Relatlimab light chain second polypeptide (SEQ ID NO: 197). Abs-513 functional antibody is assembled by introducing a polynucleotide sequence (SEQ ID NO: 277) vector expressing Abs-513 first polypeptide and a polynucleotide sequence (SEQ ID NO: 275) vector expressing Abs-513 second polypeptide into a host cell for expression

In certain embodiments, the functional antibody Abs-514 is composed of a chimeric antibody Abs-514 first polypeptide (SEQ ID NO: 200) (formed by CD80 IgV mutant E35N/A71Q via a linker peptide (SEQ ID NO: 95) covalently linked to Relatlimab antibody heavy chain N-terminus) and Relatlimab light chain second polypeptide (SEQ ID NO: 197). Abs-514 functional antibody is assembled by introducing a polynucleotide sequence (SEQ ID NO: 278) vector expressing Abs-514 first polypeptide and a polynucleotide sequence (SEQ ID NO: 276) vector expressing Abs-514 second polypeptide into a host cell for expression.

In certain embodiments, the functional antibody Abs-515 is composed of (formed by CD80 IgV mutant E35N/A71T via a linker peptide (SEQ ID NO: 95) covalently linked to Relatlimab antibody heavy chain N-terminus) a chimeric antibody Abs-515 first polypeptide (SEQ ID NO: 201) and Relatlimab light chain second polypeptide (SEQ ID NO: 197). Abs-515 functional antibody is assembled by introducing a polynucleotide sequence (SEQ ID NO: 279) vector expressing Abs-515 first polypeptide and a polynucleotide sequence (SEQ ID NO: 276) vector expressing Abs-515 second polypeptide into a host cell for expression.

In certain embodiments, the functional antibody Abs-506 is composed of a chimeric antibody Abs-506 first polypeptide (SEQ ID NO: 202) (formed by wild-type CD80 IgV via a linker peptide (SEQ ID NO: 95) covalently linked to Relatlimab antibody heavy chain N-terminus) and Relatlimab light chain second polypeptide (SEQ ID NO: 197). Abs-506 functional antibody is assembled by introducing a polynucleotide sequence (SEQ ID NO: 280) vector expressing Abs-506 first polypeptide and a polynucleotide sequence (SEQ ID NO: 276) vector expressing Abs-506 second polypeptide into a host cell for expression.

In certain embodiments, the functional antibody Abs-507 is composed of a chimeric antibody Abs-506 first polypeptide (SEQ ID NO: 204) (formed by CD80 IgV mutant E35N/A71E via a linker peptide (SEQ ID NO: 95) covalently linked to CD3B219 antibody heavy chain N-terminus) and CD3B219 light chain second polypeptide (SEQ ID NO: 203). Abs-507 functional antibody is assembled by introducing a polynucleotide sequence (SEQ ID NO: 282) vector expressing Abs-507 first polypeptide and a polynucleotide sequence (SEQ ID NO: 281) vector expressing Abs-507 second polypeptide into a host cell for expression.

In certain embodiments, the functional antibody Abs-516 is composed of (formed by CD80 IgV mutant E35N/A71N via a linker peptide (SEQ ID NO: 95) covalently linked to CD3B219 antibody heavy chain N-terminus) a chimeric antibody Abs-516 first polypeptide (SEQ ID NO: 205) and CD3B219 light chain second polypeptide (SEQ ID NO: 203). Abs-516 functional antibody is assembled by introducing a polynucleotide sequence (SEQ ID NO: 283) vector expressing Abs-516 first polypeptide and a polynucleotide sequence (SEQ ID NO: 281) vector expressing Abs-516 second polypeptide into a host cell for expression.

In certain embodiments, the functional antibody Abs-517 is composed of (formed by CD80 IgV mutant E35N/A71Q via a linker peptide (SEQ ID NO: 95) covalently linked to CD3B219 antibody heavy chain N-terminus) a chimeric antibody Abs-517 first polypeptide (SEQ ID NO: 206) and CD3B219 light chain second polypeptide (SEQ ID NO: 203). Abs-517 functional antibody is assembled by introducing a polynucleotide sequence (SEQ ID NO: 284) vector expressing Abs-517 first polypeptide and a polynucleotide sequence (SEQ ID NO: 281) vector expressing Abs-517 second polypeptide into a host cell for expression.

In certain embodiments, the functional antibody Abs-518 is composed of a chimeric antibody Abs-518 first polypeptide (SEQ ID NO: 207) (formed by CD80 IgV mutant E35N/A71T via a linker peptide (SEQ ID NO: 95) covalently linked toCD3B219 antibody heavy chain N-terminus) and CD3B219 light chain second polypeptide (SEQ ID NO: 203). Abs-518 functional antibody is assembled by introducing a polynucleotide sequence (SEQ ID NO: 285) vector expressing Abs-518 first polypeptide and a polynucleotide sequence (SEQ ID NO: 281) vector expressing Abs-518 second polypeptide into a host cell for expression

In certain embodiments, the functional antibody Abs-508 is composed of a chimeric antibody Abs-508 first polypeptide (SEQ ID NO: 207) (formed by wild-type CD80 IgV via a linker peptide (SEQ ID NO: 95) covalently linked to CD3B219 antibody heavy chain N-terminus) and CD3B219 light chain second polypeptide (SEQ ID NO: 204). Abs-508 functional antibody is assembled by introducing a polynucleotide sequence (SEQ ID NO: 286) vector expressing Abs-first polypeptide and a polynucleotide sequence (SEQ ID NO: 281) vector expressing Abs-508 second polypeptide into a host cell for expression.

In certain embodiments, the functional antibody Abs-16 is composed of a chimeric antibody Abs-16 first polypeptide (SEQ ID NO: 287) (formed by the N-terminus of the sugemalimab antibody heavy chain) and sugemalimab light chain second polypeptide (SEQ ID NO: 263). Abs-16 functional antibody is assembled by introducing a polynucleotide sequence (SEQ ID NO: 288) vector expressing Abs-16 first polypeptide and a polynucleotide sequence (SEQ ID NO: 215) vector expressing Abs-16 second polypeptide into a host cell for expression

In certain embodiments, the functional antibody Abs-58 is composed of a chimeric antibody Abs-58 first polypeptide (SEQ ID NO: 289) (formed by Relatlimab antibody heavy chain N-terminus) and Relatlimab light chain second polypeptide (SEQ ID NO: 197). Abs-58 functional antibody is assembled by introducing a polynucleotide sequence (SEQ ID NO: 290) vector expressing Abs-58 first polypeptide and a polynucleotide sequence (SEQ ID NO: 276) vector expressing Abs-58 second polypeptide into a host cell for expression.

In certain embodiments, the functional antibody Abs-519 is composed of a chimeric antibody Abs-520 first polypeptide (SEQ ID NO: 291) (formed by CD3B219 antibody heavy chain N-terminus) and CD3B219 light chain second polypeptide (SEQ ID NO: 204). Abs-519 functional antibody is assembled by introducing a polynucleotide sequence (SEQ ID NO: 292) vector expressing Abs-519 first polypeptide and a polynucleotide sequence (SEQ ID NO: 281) vector expressing Abs-519 second polypeptide into a host cell for expression.

In certain embodiments, the functional antibody Abs-520 is composed of a chimeric antibody Abs-520 first polypeptide (SEQ ID NO: 293) (formed by CD80 IgV mutant E35N/A71E via a linker peptide (SEQ ID NO: 95) covalently linked to the N-terminus of the sugemalimab antibody heavy chain and sugemalimab antibody heavy chain C-terminus via a linker peptide (SEQ ID NO: 95) covalently linked to LAG3 polypeptide) and sugemalimab light chain second polypeptide (SEQ ID NO: 141). Abs-520 multifunctional antibody is assembled by introducing a polynucleotide sequence (SEQ ID NO: 294) vector expressing Abs-520 first polypeptide and a polynucleotide sequence (SEQ ID NO: 215) vector expressing Abs-520 second polypeptide into a host cell for expression.

In certain embodiments, the functional antibody Abs-521 is composed of a chimeric antibody Abs-521 first polypeptide (SEQ ID NO: 295) (formed by CD80 IgV mutant E35N/A71N via a linker peptide (SEQ ID NO: 95) covalently linked to the N-terminus of the sugemalimab antibody heavy chain and sugemalimab antibody heavy chain C-terminus via a linker peptide (SEQ ID NO: 95) covalently linked toLAG3 polypeptide) and sugemalimab light chain second polypeptide (SEQ ID NO: 141). Abs-521multifunctional antibody is assembled by introducing a polynucleotide sequence (SEQ ID NO: 296) vector expressing Abs-520 first polypeptide and a polynucleotide sequence (SEQ ID NO: 215) vector expressing Abs-521 second polypeptide into a host cell for expression.

Although various embodiments of the present invention have been described above, it should be understood that they have been presented by way of example only, and not limitation. Many modifications to the disclosed embodiments may be made in light of the invention herein without departing from the spirit or scope of the invention. Thus, the breadth and scope of the present invention should not be limited by any of the above-described embodiments.

All documents mentioned in this invention are incorporated herein by reference. All publications and patent documents cited in this application are incorporated by reference for all purposes to the same extent as if each individual publication or patent document were individually denoted.

### Example

### Example 1. Antibody expression and purification

In order to further identify the screened antibodies, it is necessary to express the antibodies in mammalian cells. Therefore, firstly, an expression plasmid vector for expressing the heavy chain of the multi/bifunctional antibody and an expression plasmid vector containing the light chain were constructed. About 24 h before plasmid transfection, Expi293 cells were passaged to a cell density of about 2.4×10⁶ cells/ml. When the cell density is 6×10⁶ cells/ml and the viability is >95%, take 25ug of the plasmid vector expressing the mixed heavy chain and light chain and transfect 25ml of Expi293 cells using Expifectamine 293, and culture at 37°C, 130rpm, 8% CO2 in a shaking incubator for 7 days. The cell culture product is centrifuged and the supernatant is taken. After filtering with a 0.45 MCE filter, the target antibody is purified and collected using a Capturem protein A Maxiprep column, and then concentrated by centrifugation with a Vivaspin 20 Centrifugal Concentrator 50K, quantified by the A280 determination method using NanoDrop 2000, and the antibody purity is determined by SDS-PAGE and SEC-HPLC.

### Example 2. Protein electrophoresis analysis

The following CD80 IgV mutant-sugemalimab fusion polypeptide complexes selected and purified under reducing conditions (N-R) and non-reducing conditions (N) were analyzed by SDS PAGE: Abs-99, Abs-0247, Abs-248, Abs-249, Abs-250, Abs-251, Abs-252, Abs-253, Abs-254, Abs-255, Abs-256, Abs-257, Abs-258, Abs-259, Abs-260, Abs-261, Abs-262, Abs-263, Abs-264, Abs-265, Abs-266, Abs-267, Abs-268, Abs-269, Abs-300, Abs-311, Abs-312, Abs-313, Abs-314, Abs-315, Abs-316, Abs-317, Abs-318, Abs-319, Abs-320, Abs-321, Abs-322, Abs-323, Abs-324, Abs-325 bs-266, Abs-267, Abs-268, Abs-269, Abs-270, Abs-271, Abs-272, Abs-273, Abs-353, Abs-354, Abs-355, Abs-356, Abs-357, Abs-358, Abs-359, Abs-360, Abs-361, Abs-362, Abs-363, Abs-364, Abs-365, Abs-366, Abs-367, Abs-368.

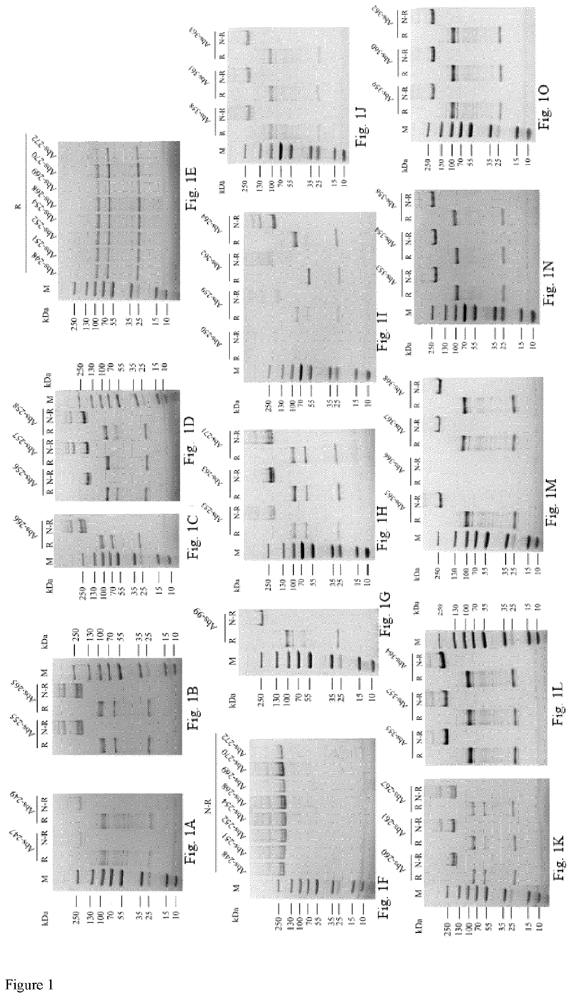

The results of electrophoresis analysis showed that the CD80 IgV mutant-sugemalimab fusion polypeptide complex Abs-353, Abs-354, Abs-355, Abs-356, Abs-357, Abs-360, Abs-362, Abs-364, Abs-367, Abs-368 and other antibody assemblies were superior to the wild-type CD80 IgV-sugemalimab fusion polypeptide complex Abs-99, as shown in FIG. 1 .

### Example 3. Binding affinity determination

This example shows the binding of the CD80 mutant fusion polypeptide complex to human PD-L1 protein, human CTLA4 protein and human CD28.

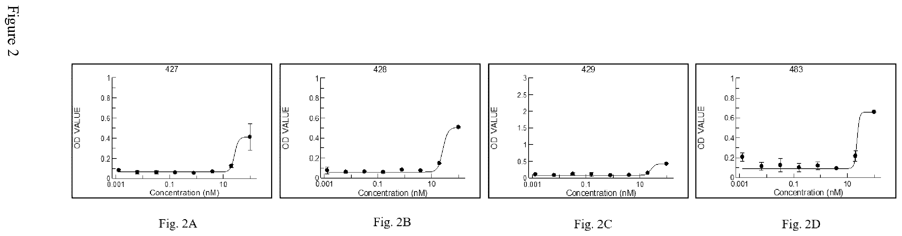

Specifically, human PD-L1 protein (1 µg/mL in PBS; Acro, PD1-H5258) was incubated in a flat-bottomed 96-well Immuno detachable transparent ELISA plate (MaxiSorp) (Thermo Scientific, 446469) at 4°C overnight; then, after washing, 2% BSA (in PBS; VWR Life Science, 0332-1KG) was added to each well and incubated at room temperature for 1 hour, after washing, serial dilutions of the test substance were added to each well and added to the final test concentration and incubated at room temperature for 2 hours, after washing, Goat Anti-Human Lambda-HRP (Southern Biotech, 2060-05) was added to each well and incubated at room temperature for 30 minutes, after washing, **the display** was detected using the Soluble TMB Kit (CW0050S), and the specific operations were performed according to the manufacturer's instructions. The affinity of the candidate multi/bifunctional fusion proteins to the human PD-L1 protein was expressed by EC50 and 20 nM binding intensity. The test results are shown in Table 1 and Figure 2.

**Table 1. EC50 and binding strength at 100 nM of CD80 mutant fusion polypeptide complexes binding to human PDL1 protein**

| Antibody ID | PD-L1 ELISA binding | |
|---|---|---|
| | EC50(nM) | Binding strength @ 100nM |
| Wildtype Abs-504 | NA | |
| Abs-427 | 26.3654 | 400% |
| Abs-428 | 26.9074 | 400% |
| Abs-429 | NA | |
| Abs-483 | 22.8881 | 700% |

The results shown in Table 1 and Figure 2 indicate that in the CD80 mutant fusion polypeptide complex binding experiment with human PDL1 antigen, the CD80 mutant fusion polypeptide complexes Abs-427, Abs-428, and Abs-483 have much higher binding activity with PDL1 antigen than the wild-type CD80 fusion polypeptide complex Abs-504.

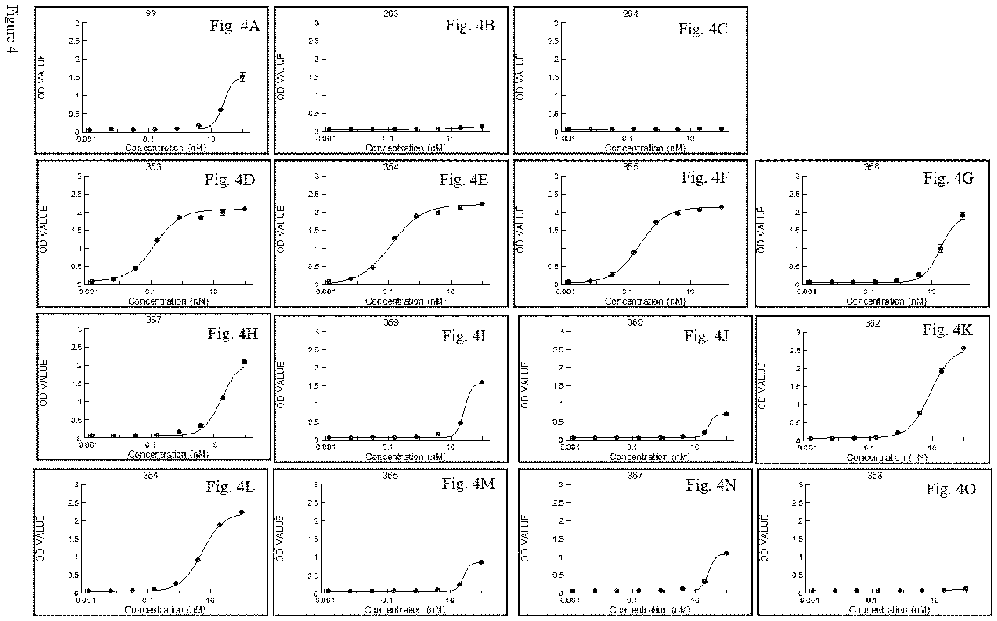

Similarly, human CD28 protein (1 µg/mL in PBS; Acro Corporation, CD8-H525a) was incubated in a flat-bottomed 96-well Immuno detachable transparent ELISA plate (MaxiSorp) (Thermo Scientific, 446469) at 4°C overnight; the affinity of the test substance to the human CD28 protein was detected using a similar method as described above, and the affinity was expressed as EC50 and a binding strength of 100 nM. The test results are shown in Table 2 and Figure 4.

**Table 2. EC50 and binding strength at 100 nM of CD80 mutant fusion polypeptide complexes binding to human CD28 protein**

| Antibody ID | CD28 ELISA binding | |
|---|---|---|
| | EC50(nM) | binding strength @ 100nM |
| wild type Abs-99 | 27.93 | 283% |
| Abs-247 | NA | |
| Abs-248 | NA | |
| Abs-249 | NA | |
| Abs-250 | NA | |
| Abs-251 | NA | |
| Abs-252 | NA | |
| Abs-253 | NA | |
| Abs-254 | NA | |
| Abs-255 | NA | |
| Abs-256 | NA | |
| Abs-257 | NA | |
| Abs-258 | NA | |
| Abs-259 | NA | |
| Abs-260 | NA | |
| Abs-261 | NA | |
| Abs-262 | NA | |
| Abs-263 | 26.13 | 333% |
| Abs-264 | NA | |
| Abs-265 | NA | |
| Abs-266 | NA | |
| Abs-267 | NA | |
| Abs-268 | NA | |
| Abs-269 | NA | |
| Abs-270 | NA | |
| Abs-271 | NA | |
| Abs-272 | NA | |
| Abs-273 | NA | |
| Abs-353 | 25.01 | 1450% |
| Abs-354 | 22.05 | 6300% |
| Abs-355 | 26.79 | 530% |
| Abs-356 | 27.79 | 733% |
| Abs-357 | 26.01 | 585% |
| Abs-358 | NA | |
| Abs-359 | 21.06 | 450% |
| Abs-360 | 25.9 | 933% |
| Abs-361 | NA | |
| Abs-362 | 26.13 | 1100% |
| Abs-363 | NA | |
| Abs-364 | 27.6 | 785% |
| Abs-365 | 27.89 | 857% |
| Abs-366 | NA | |
| Abs-367 | 27.74 | 700% |
| Abs-368 | 28.71 | 700% |

| | | |
|---|---|---|
| NA: indicates no binding to the target protein | | |

The results shown in Table 2 and Figure 4 indicate that in the CD80 mutant fusion polypeptide complex binding experiment with human CD28 antigen, the antigen binding activity of the CD80 mutant fusion polypeptide complexes Abs-353, Abs-354, Abs-355, Abs-356, Abs-357, Abs-359, Abs-360, Abs-362, Abs-364, Abs-365, Abs-367, and Abs-368 to CD28 is much higher than that of the wild-type CD80 fusion polypeptide complex Abs-99.

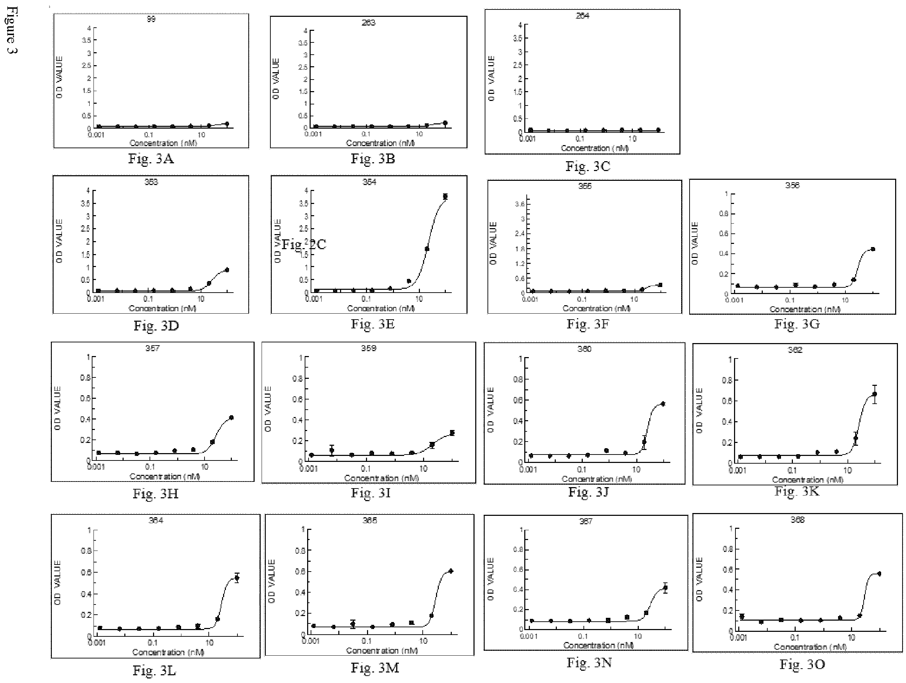

Similarly, human CTLA4 protein (1 µg/mL in PBS; Acro, CT4-H52H9) was incubated in a flat-bottomed 96-well Immuno detachable transparent ELISA plate (MaxiSorp) (Thermo Scientific, 446469) at 4°C overnight; the affinity of the test substance to the human CTLA4 protein was detected using a similar method as described above, and the affinity was expressed as EC50 and a binding strength of 20 nM. The test results are shown in Table 3 and Figure 3.

**Table 3. EC50 and binding strength at 20 nM of CD80 mutant fusion polypeptide complexes binding to human CTLA4 protein**

| Antibody ID | CTLA4 ELISA binding | |
|---|---|---|
| | EC50(nM) | binding strength @ 20nM |
| wild type Abs-99 | 24.4 | 1200% |
| Abs-247 | NA | |
| Abs-248 | NA | |
| Abs-249 | NA | |
| Abs-250 | NA | |
| Abs-251 | NA | |
| Abs-252 | NA | |
| Abs-253 | NA | |
| Abs-254 | NA | |
| Abs-255 | NA | |
| Abs-256 | NA | |
| Abs-257 | NA | |
| Abs-258 | NA | |
| Abs-259 | NA | |
| Abs-260 | NA | |
| Abs-261 | NA | |
| Abs-262 | NA | |
| Abs-263 | NA | |
| Abs-264 | NA | |
| Abs-265 | NA | |
| Abs-266 | NA | |
| Abs-267 | 13.69 | 1916% |
| Abs-268 | NA | |
| Abs-269 | NA | |
| Abs-270 | NA | |
| Abs-271 | NA | |
| Abs-272 | NA | |
| Abs-273 | NA | |
| Abs-353 | 0.128 | 2487% |
| Abs-354 | 0.124 | 3014% |
| Abs-355 | 0.236 | 3417% |
| Abs-356 | 18.48 | 1667% |
| Abs-357 | 17.805 | 1833% |
| Abs-358 | NA | |
| Abs-359 | 26.45 | 766% |
| Abs-360 | 26.74 | 316% |
| Abs-361 | NA | |
| Abs-362 | 8.393 | 3183% |
| Abs-363 | NA | |
| Abs-364 | 5.449 | 3133% |
| Abs-365 | 24.69 | 400% |
| Abs-366 | NA | |
| Abs-367 | 26.47 | 640% |
| Abs-368 | NA | |

| | | |
|---|---|---|
| NA: indicates no binding to the target protein | | |

The results shown in Table 3 and Figure 3 indicate that in the CD80 mutant fusion polypeptide complex binding experiment with human CTLA4 antigen, the CD80 mutant fusion polypeptide complexes Abs-267, Abs-353, Abs-354, Abs-355, Abs-356, Abs-357, Abs-362, and Abs-364 have much higher binding activity with the CTLA4 antigen than the wild-type CD80 fusion polypeptide complex Abs-99.

### Example 4. Determination of the CD80 mutant fusion polypeptide complex to relieve PD-1/PD-L1 mediated T cell immunosuppression

This example shows that the CD80 mutant fusion polypeptide complex relieves the PD-1/PD-L1-mediated T cell inhibitory response.

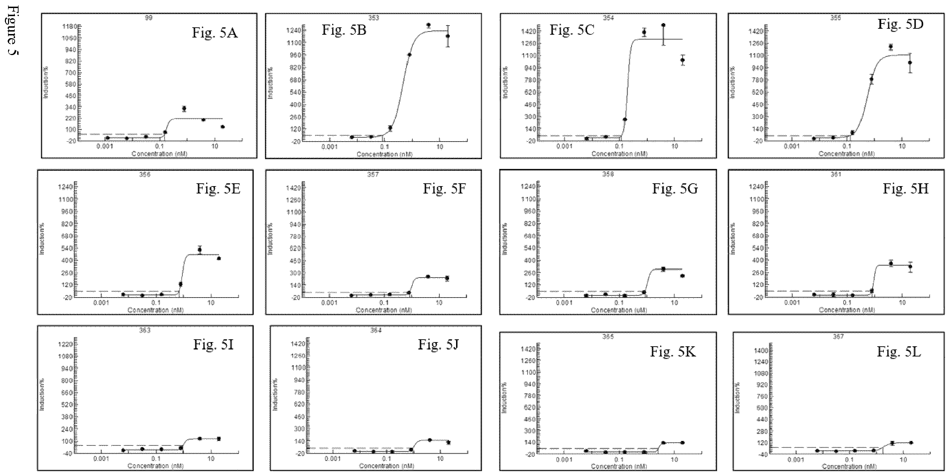

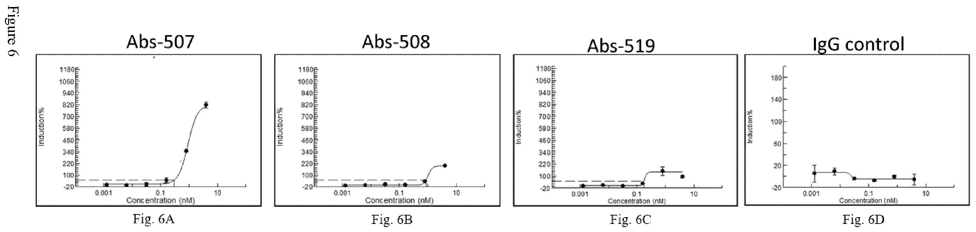

Specifically, on the first day, TCR activator PD-L1-CHO cells (BPS bioscience) were digested and collected using 0.05% trypsin, counted, and plated; fresh culture medium was added to mix the cells and the cell density was adjusted to 3.5*10⁵ cells/ml. Add cells to a sterile 96-well flat-bottom plate (CORNING, 3599) at 100ul/well, for a total of 35,000 cells/well; grow overnight in a 37°C incubator to 80%; the next day, remove the culture medium of TCR activator PD-L1-CHO cells in the 96-well flat-bottom plate, add 50ul of fresh culture medium containing the corresponding concentration of test substance (serial dilution) to each well, and after 30 minutes of treatment, collect Jurkat-Lucia^{™} TCR-hPD-1 cells (Invivogen) and count; add fresh culture medium to mix the cells and adjust the cell density to 4*10⁵ cells/ml; add 50ul Jurkat-Lucia^{™} TCR-hPD-1 cells to a final cell density of 2*10⁴/well, and incubate in a 96-well flat-bottom plate in a 37°C incubator for 5-6h. After incubation, the cell morphology was observed under a microscope and the chemiluminescence value was detected by QUANTI-Luc^{™} (Invivogen; rep-qlc1). The specific operation was carried out according to the manufacturer's instructions. The activity was expressed by EC50 and the highest induction fold. The test results are shown in Table 4, Table 5, Figure 5 and Figure 6.

**Table 4. Results of assays of CD80 mutant fusion polypeptide complexes regulating Jurkat T cell activation**

| Antibody ID | Jurkat-NFAT-Luc/CHO-PDL1 | |
|---|---|---|
| | EC50(nM) | The highest induction relative to 10 nM sugemalimab |
| wild type Abs-99 | 0.28 | 215% |
| Abs-247 | NT | |
| Abs-248 | NT | |
| Abs-249 | NT | |
| Abs-250 | NT | |
| Abs-251 | NT | |
| Abs-252 | NT | |
| Abs-253 | NT | |
| Abs-254 | NT | |
| Abs-255 | NT | |
| Abs-256 | 0.87 | 133% |
| Abs-257 | 0.82 | 122% |
| Abs-258 | NT | |
| Abs-259 | NT | |
| Abs-260 | 0.65 | 128% |
| Abs-261 | NT | |
| Abs-262 | NT | |
| Abs-263 | NT | |
| Abs-264 | 0.82 | 154% |
| Abs-265 | NT | |
| Abs-266 | NT | |
| Abs-267 | NT | |
| Abs-268 | NT | |
| Abs-269 | NT | |
| Abs-270 | NT | |
| Abs-271 | NT | |
| Abs-272 | NT | |
| Abs-273 | NT | |
| Abs-353 | 0.48 | 1133% |
| Abs-354 | 0.25 | 1610% |
| Abs-355 | 0.56 | 1107% |
| Abs-356 | 0.90 | 441% |
| Abs-357 | 1.00 | 238% |
| Abs-358 | 1.10 | 277% |
| Abs-359 | 0.88 | 42% |
| Abs-360 | 0.82 | 64% |
| Abs-361 | 1.13 | 334% |
| Abs-362 | 0.58 | 337% |
| Abs-363 | 1.00 | 121% |
| Abs-364 | 1.06 | 150% |
| Abs-365 | 2.75 | 129% |
| Abs-366 | NT | |
| Abs-367 | 1.84 | 113% |
| Abs-368 | 0.52 | 138% |

| | | |
|---|---|---|
| NT: indicates not tested, NA: indicates no agonist effect | | |

The results in Table 4 and Figure 5 show that in the experiment of regulating Jurkat T cell activation by CD80 mutant fusion polypeptide complexes, the ability of CD80 mutant fusion polypeptide complexes Abs-353, Abs-354, Abs-355, Abs-356, Abs-357, Abs-358, Abs-361, and Abs-362 in regulating Jurkat T cell activation was higher than that of the wild-type CD80 fusion polypeptide complex Abs-99.

**Table 5. Results of assays of CD80 mutant fusion polypeptide complexes regulating Jurkat T cell activation**

| Antibody ID | Jurkat-NFAT-Luc/CHO-PDL1 | |
|---|---|---|
| | EC50(nM) | The highest induction relative to IgG control |
| wild type Abs-504 | NA | |
| Abs-427 | NA | |
| Abs-428 | 4.2 | 200% |
| Abs-429 | NA | |
| Abs-483 | NA | |

| | | |
|---|---|---|
| NA: indicates no agonist effect | | |

The results in Table 5 and Figure 6 show that in the experiment of CD80 mutant fusion polypeptide complex regulating Jurkat T cell activation, the ability of CD80 mutant fusion polypeptide complex Abs-428 to regulate Jurkat T cell activation is higher than that of wild-type CD80 fusion polypeptide complex Abs-504.

### Example 5. Determination of CD80 mutant fusion polypeptide complexes to relieve LAG3-mediated T cell immunosuppression

This example shows that the CD80 mutant fusion polypeptide complex relieves LAG3-mediated T cell inhibitory response.

Specifically, Raji cells (ATCC) were collected, counted and plated. Add fresh culture medium to mix the cells and adjust the cell density to 3.5*10⁵ cells/ml. The cells were added to a sterile 96-well flat-bottom plate (CORNING, 3599) at 100 ul/well, for a total of 35,000 cells/well. 50ul of fresh culture medium containing the corresponding concentration of test substances (serially diluted) was added to each well. After 30min of treatment, LAG3/NFAT Reporter-Jurkat cells (BPS bioscience) were collected and counted. Fresh culture medium containing 0.4 ng/ml Staphylococcal enterotoxin E (SEE, Toxintechnology) was added to mix the cells and adjust the cell density to 4*10⁵ **cells/m**l. Add 50ul LAG3 / NFAT Reporter - Jurkat cells to a final cell density of 2*10⁴ /well and incubate in a 96-well flat-bottom plate in a 37°C incubator for 5-6h. After incubation, cell morphology was observed under a microscope and chemiluminescence was detected using QUANTI-Luc^{™} (BPS bioscience; 78262) according to the manufacturer's instructions.

**Table 6. Results of the assay to determine whether CD80 mutant fusion polypeptide complex relieves LAG3-mediated T cell immunosuppression**

| Antibody ID | LAG3 / NFAT Reporter-Jurkat/Raji | |
|---|---|---|
| | EC50(nM) | The highest induction relative to IgG control |
| Abs-505 | 2.4 | 602% |
| Abs-513 | 1.8 | 626% |
| Abs-514 | 2.0 | 500% |
| Abs-515 | 3.14 | 352% |
| wild type Abs-506 | 2.5 | 220% |
| Abs-58 | 2.9 | 218% |

In Table 6, the experiment of CD80 mutant fusion polypeptide complexes relieving LAG3-mediated T cell immunosuppression reaction shows that the CD80 mutant fusion polypeptide complexes Abs-505, Abs-513, Abs-514, and Abs-515 have higher ability to relieve LAG3-mediated T cell immunosuppression than the wild-type CD80 fusion polypeptide complex Abs-506.

### Example 6. T cell activation immune response assay

This example shows that the CD80 mutant fusion polypeptide complex regulates T cell immune response.

Specifically, Jurkat-Lucia^{™} TCR cells (Invivogen) were collected and counted. Add fresh culture medium to mix the cells and adjust the cell density to 4*10⁵ cells/ml. 100 ul of fresh culture medium containing the test substance (serial dilutions) at the corresponding concentration was added to each well and incubated in a 96-well flat-bottom plate in a 37°C incubator for 5-6 h. After incubation, cell morphology was observed under a microscope and chemiluminescence was detected using QUANTI-Luc^{™} (Invivogen; rep-qlc1) according to the manufacturer's instructions.

**Table 7. Results of determination of T cell activated immune response of CD80 mutant fusion polypeptide complexes**

| Antibody ID | Jurkat-Lucia^{™} TCR | |
|---|---|---|
| | EC50(nM) | The highest induction relative to IgG control |
| Abs-507 | 0.51 | 814% |
| Abs-516 | 0.82 | 756% |
| Abs-517 | 0.35 | 783% |
| Abs-518 | 0.56 | 429% |
| wild type Abs-508 | 0.31 | 195% |
| Abs-519 | 0.19 | 141% |

The results in Table 7 show that in the CD80 mutant fusion polypeptide complex T cell activation immune response assay, the CD80 mutant fusion polypeptide complexes Abs-507, Abs-516, Abs-517, and Abs-518 have higher abilities to activate T cell immune responses than the wild-type CD80 fusion polypeptide complex Abs-508.

### Example 7. Primary PBMC immune response assay

This example shows that CD80 mutant fusion polypeptide complexes activate T cell immunity in primary PBMCs. Specifically, PBMCs were collected, counted, and then plated. Add fresh culture medium to mix the cells evenly and adjust the cell density to 1*10⁵ cells/ml. 50ul of fresh culture medium containing the test substance (serial dilutions) of corresponding concentration was added to each well. After 3 days of incubation, the culture supernatant was collected and the secretion of IL-2 in the supernatant was detected using an IL-2 ELISA detection kit (R&D, DY202). The specific operation was carried out according to the manufacturer's instructions.

**Table 8. Results of determination of CD80 mutant fusion polypeptide complexes activating primary PBMC T cell immune responses**

| Antibody ID | PBMC | |
|---|---|---|
| | EC50(nM) | The highest induction relative to IgG control |
| Abs-353 | 1.78 | 351% |
| Abs-354 | 1.19 | 389% |
| Abs-355 | 1.59 | 310% |
| Abs-356 | 1.24 | 239% |
| wild type Abs-99 | 1.38 | 201% |

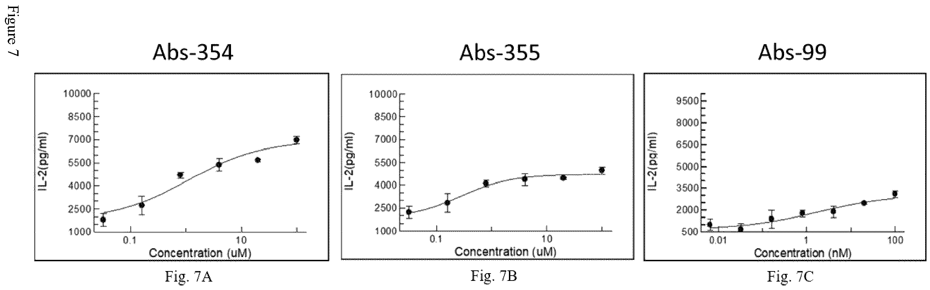

The results in Table 8 and Figure 7 show that the CD80 mutant fusion polypeptide complexes Abs-353, Abs-354, Abs-355, and Abs-356 have higher abilities to activate T cells in primary PBMCs than the wild-type CD80 fusion polypeptide complex Abs-99.

### Example 8. Determination of Antigen Presenting Cells Secreting Chemokine

This example shows that Abs-induced antigen presenting cells secrete the chemokine CCL4.

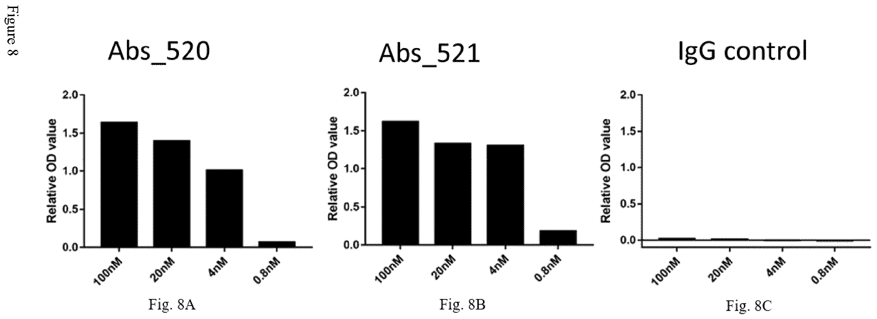

Specifically, antigen presenting cells THP-1 cells (ATCC) were collected, counted and plated. Add fresh culture medium to mix the cells evenly and adjust the cell density to 1*10⁶ cells/ml, and plate 100ul of cells in each well. 100ul of fresh culture medium containing the corresponding concentration of the test substance Abs_520 or Abs_521 was added to each well. After 6 hours, the cell culture supernatant was collected and the expression level of CCL4 was detected using a CCL4 detection kit (PROTEINTECH). The specific operation was carried out according to the manufacturer's instructions. The results showed that Abs_520 and Abs_521 could induce antigen presenting cells to secrete chemokines, as shown in FIG8 .

The results of the above examples demonstrate that the CD80 mutant polypeptide formed by innovatively modifying the CD80 IgV domain can regulate the binding activity of CD80 to its ligand. Specifically, the antigen binding activity of the CD80 mutant fusion polypeptide complex disclosed herein to PDL1, CTLA4, and CD28 is significantly higher than that of the corresponding wild-type CD80 fusion polypeptide complex; the CD80 mutant fusion polypeptide complex disclosed herein can relieve the PD-1/PD-L1-mediated T cell activation immunosuppression reaction; can regulate Jurkat T cell activation; can regulate PBMC activation; and promote antigen presenting cells to secrete chemokines. That is, the CD80 mutant fusion polypeptide complex disclosed herein can effectively excite T cells and enhance immune response, thereby achieving the treatment or prevention of diseases such as infection and tumor caused by the inhibition of T cell function.

Although various embodiments of the present invention have been described above, it should be understood that they have been presented by way of example only, and not limitation. Without departing from the spirit and scope of the present invention, the present invention may be subject to various changes and modifications, which will fall within the scope of the present invention as claimed. The scope of protection sought for the present invention is defined by the following claims and their equivalents.

## Claims

1. A CD80 IgV mutant polypeptide, which, relative to the wild-type human CD80 IgV polypeptide sequence SEQ ID NO: 45, comprises one or more amino acid mutations selected from the group consisting of T13K, A26M, A26C, E35N, E35S, E35Q, E35T, M42L, M47Q, M47H, M47K, M47T, I69Y, I69L, I69W, A71E, A71N, A71Q, A71E, V83L, L85N, and L85D.

2. The CD80 IgV mutant polypeptide of claim 1, wherein the CD80 IgV mutant polypeptide comprises a combination of two or more of the amino acid mutations, optionally wherein the combination of two or more of the amino acid mutations is selected from E35N/A71E (SEQ ID NO: 46), E35N/A71N (SEQ ID NO: 47), E35N/A71Q (SEQ ID NO: 48), E35N/A71T (SEQ ID NO: 49), E35S/A71E (SEQ ID NO: 50), E35S/A71N (SEQ ID NO: 51), E35S/A71Q (SEQ ID NO: 52), E35S/A71T (SEQ ID NO: 53), E35Q/A71E (SEQ ID NO: 54), E35Q/A71N (SEQ ID NO: 55), E35Q/A71Q (SEQ ID NO: 56), E35Q/A71T (SEQ ID NO: 57), E35T/A71E (SEQ ID NO: 58), E35T/A71N (SEQ ID NO: 59), E35T/A71Q (SEQ ID NO: 60), E35T/A71T (SEQ ID NO: 61), M47A/I69Y/A71E/V83L (SEQ ID NO: 62), M47A/I69W/A71E/V83L (SEQ ID NO: 63), M47A/I69Y/A71E (SEQ ID NO: 64), M47A/I69W/A71E (SEQ ID NO: 65), I69Y/A71E/V83L (SEQ ID NO: 66), I69W/A71E/V83L (SEQ ID NO: 67), I69Y/A71E (SEQ ID NO: 68), I69W/A71E (SEQ ID NO: 69), H18W/A26M/A71E/L85N (SEQ ID NO: 70), H18W/A26M/A71E/L85D (SEQ ID NO: 71), H18W/A26C/A71E/L85N (SEQ ID NO: 72), H18W/A26C/A71E/L85D (SEQ ID NO: 73), A26M/A71E/L85N (SEQ ID NO: 74), A26M/A71E/L85D (SEQ ID NO: 75), A26C/A71E/L85D (SEQ ID NO: 76), A26C/A71E/L85N (SEQ ID NO: 77), I69L/A71E/V83L (SEQ ID NO: 78), H18W/A26V/E35N/A71E/L85N (SEQ ID NO: 79), T13K/M42L/M47H/A71E (SEQ ID NO: 80), T13K/M42L/M47Q/A71E (SEQ ID NO: 81), T13K/M42L/A71E (SEQ ID NO: 82), T13K/M42T/M47H/A71E (SEQ ID NO: 83), T13K/M42T/M47T/A71E (SEQ ID NO: 84), T13K/M42T/A71E (SEQ ID NO: 85), M47H/I69Y/A71E/V83L (SEQ ID NO: 86), M47Q/I69Y/A71E/V83L (SEQ ID NO: 87) and M47K/I69Y/A71E/V83L (SEQ ID NO: 88).

3. A CD80 ECD mutant polypeptide, which, relative to the wild-type human CD80 ECD polypeptide sequence SEQ ID NO: 1, comprises one or more amino acid mutations selected from the group consisting of T13K, A26M, A26C, E35N, E35S, E35Q, E35T, M42L, M47Q, M47H, M47K, M47T, I69Y, I69L, I69W, A71E, A71N, A71Q, A71E, V83L, L85N, and L85D.

4. The CD80 ECD mutant polypeptide of claim 3, wherein the CD80 ECD mutant polypeptide comprises a combination of two or more of the amino acid mutations, optionally wherein the combination of two or more of the amino acid mutations is selected from E35N/A71E (SEQ ID NO: 2), E35N/A71N (SEQ ID NO: 3), E35N/A71Q (SEQ ID NO: 4), E35N/A71T (SEQ ID NO: 5), E35S/A71E (SEQ ID NO: 6), E35S/A71N (SEQ ID NO: 7), E35S/A71Q (SEQ ID NO: 8), E35S/A71T (SEQ ID NO: 9), E35Q/A71E (SEQ ID NO: 10), E35Q/A71N (SEQ ID NO: 11), E35Q/A71Q (SEQ ID NO: 12), E35Q/A71T (SEQ ID NO: 13), E35T/A71E (SEQ ID NO: 14), E35T/A71N (SEQ ID NO: 15), E35T/A71Q (SEQ ID NO: 16), E35T/A71T (SEQ ID NO: 17), M47A/I69Y/A71E/V83L (SEQ ID NO: 18), M47A/I69W/A71E/V83L (SEQ ID NO: 19), M47A/I69Y/A71E (SEQ ID NO: 20), M47A/I69W/A71E (SEQ ID NO: 21), I69Y/A71E/V83L (SEQ ID NO: 22), I69W/A71E/V83L (SEQ ID NO: 23), I69Y/A71E (SEQ ID NO: 24), I69W/A71E (SEQ ID NO: 25), H18W/A26M/A71E/L85N (SEQ ID NO: 26), H18W/A26M/A71E/L85D (SEQ ID NO: 27), H18W/A26C/A71E/L85N (SEQ ID NO: 28), H18W/A26C/A71E/L85D (SEQ ID NO: 29), A26M/A71E/L85N (SEQ ID NO: 30), A26M/A71E/L85D (SEQ ID NO: 31), A26C/A71E/L85D (SEQ ID NO: 32), A26C/A71E/L85N (SEQ ID NO: 33), I69L/A71E/V83L (SEQ ID NO: 34), H18W/A26V/E35N/A71E/L85N (SEQ ID NO: 35), T13K/M42L/M47H/A71E (SEQ ID NO: 36), T13K/M42L/M47Q/A71E (SEQ ID NO: 37), T13K/M42L/A71E (SEQ ID NO: 38), T13K/M42T/M47H/A71E (SEQ ID NO: 39), T13K/M42T/M47T/A71E (SEQ ID NO: 40), T13K/M42T/A71E (SEQ ID NO: 41), M47H/I69Y/A71E/V83L (SEQ ID NO: 42), M47Q/I69Y/A71E/V83L (SEQ ID NO: 43) and M47K/I69Y/A71E/V83L (SEQ ID NO: 44).

5. A CD80 mutant fusion polypeptide complex, comprising the CD80 IgV mutant polypeptide of claim 1 or 2 or the CD80 ECD mutant polypeptide of claim 3 or 4 and a second domain, optionally wherein the CD80 IgV mutant polypeptide or the CD80 ECD mutant polypeptide is covalently linked to the second domain with or without a linker peptide.

6. The CD80 mutant fusion polypeptide complex of claim 5, wherein the second domain is an antibody or an antigen-binding fragment thereof, optionally wherein the antigen-binding fragment is selected from Fab, Fab', Fv, F(ab)2, F(ab')2, scFv, VHH, di-scFv and/or dAb.

7. The CD80 mutant fusion polypeptide complex of claim 6, wherein the antibody is selected from an immunoglobulin IgG antibody, a recombinant antibody, a chimeric antibody, a heavy chain antibody, a single domain antibody and/or a bispecific antibody, optionally, wherein the immunoglobulin IgG antibody is selected from human IgG1, human IgG2, human IgG3, human IgG4, mouse IgG1, mouse IgG2a, mouse IgG2b or mouse IgG3.

8. The CD80 mutant fusion polypeptide complex of claim 6 or 7, wherein the antibody or antigen-binding fragment thereof specifically targets one or more antigens selected from the group consisting of PD-L1, PD-L2, PD-1, OX40, 4-1BB, ICOS, TIGIT, CTLA4, LAG3, CD3, VEGF, VEGFR, CD47, HGF, Trop2, EpCAM, CCR8, CCR4, CCR5, GPRC5D, BCMA, CD19, CD20, HER-2 neu, DLL1, HER-3, HER-4, EGFR, PSMA, CEA, MUC-1 (mucin), MUC2, MUC3, MUC4, MUC5AC, MUC5B, MUC7, CD123, CD33, CD30, CD38, NKG2A, Nkp36 and/or Tim3.

9. The CD80 mutant fusion polypeptide complex of claim 8, wherein the antibody or antigen-binding fragment thereof specifically targets PD-L1, PD-1, TIGIT, CTLA4, LAG3 or CD3.

10. The CD80 mutant fusion polypeptide complex of claim 9, wherein the antibody or antigen-binding fragment thereof targeting PD-L1 is Sugemalimab antibody or antigen-binding fragment thereof; the antibody or antigen-binding fragment thereof targeting LAG-3 is Relatlimab antibody or antigen-binding fragment thereof; and/or the antibody or antigen-binding fragment thereof targeting CD3 is CD3B219 antibody or antigen-binding fragment thereof.

11. The CD80 mutant fusion polypeptide complex of any one of claims 6 to 9, wherein the antibody or antigen-binding fragment thereof comprises a heavy chain, and the CD80 IgV mutant polypeptide or the CD80 ECD mutant polypeptide is covalently linked to the heavy chain with or without a linker peptide, optionally, wherein the C-terminus of the CD80 IgV mutant polypeptide or the CD80 ECD mutant polypeptide is covalently linked to the N-terminus of the heavy chain with or without a linker peptide; and/or the C-terminus of the heavy chain is covalently linked to the N-terminus of the CD80 IgV mutant polypeptide or the CD80 ECD mutant polypeptide with or without a linker peptide.

12. The CD80 mutant fusion polypeptide complex of any one of claims 6 to 10, wherein the antibody or antigen-binding fragment thereof comprises a light chain, and the CD80 IgV mutant polypeptide or the CD80 ECD mutant polypeptide is covalently linked to the light chain with or without a linker peptide, optionally, wherein the C-terminus of the CD80 IgV mutant polypeptide or the CD80 ECD mutant polypeptide is covalently linked to the N-terminus of the light chain with or without a linker peptide; and/or the C-terminus of the light chain is covalently linked to the N-terminus of the CD80 IgV mutant polypeptide or the CD80 ECD mutant polypeptide with or without a linker peptide.

13. The CD80 mutant fusion polypeptide complex of claim 5, wherein the CD80 mutant fusion polypeptide complex comprises a first polypeptide and a second polypeptide, and the second polypeptide may be present or absent, optionally wherein the first polypeptide comprises a heavy chain of an antibody or an antigen-binding fragment thereof, and/or the second polypeptide comprises a light chain of an antibody or an antigen-binding fragment thereof.

14. The CD80 mutant fusion polypeptide complex of claim 13, wherein the first polypeptide is covalently linked in sequence from the N-terminus to the C-terminus of the polypeptide by a CD80 IgV mutant polypeptide or a CD80 ECD mutant polypeptide, a linker peptide, and a heavy chain of an antibody or an antigen-binding fragment thereof, or the first polypeptide is covalently linked in sequence from the N-terminus to the C-terminus of the polypeptide by a heavy chain of an antibody or an antigen-binding fragment thereof, a linker peptide, a CD80 IgV mutant polypeptide or a CD80 ECD mutant polypeptide.

15. The CD80 mutant fusion polypeptide complex of claim 14, wherein the second polypeptide is covalently linked in sequence from the N-terminus to the C-terminus of the polypeptide by a CD80 IgV mutant polypeptide or a CD80 ECD mutant polypeptide, a linker peptide, and a light chain of an antibody or an antigen-binding fragment thereof; or the second polypeptide is covalently linked in sequence from the N-terminus to the C-terminus of the polypeptide by a light chain of an antibody or an antigen-binding fragment thereof, a linker peptide, a CD80 IgV mutant polypeptide or a CD80 ECD mutant polypeptide.

16. The CD80 mutant fusion polypeptide complex of claim 13 or 14, wherein the first polypeptide is selected from E35N/A71E (SEQ ID NO: 97), E35N/A71N (SEQ ID NO: 98), E35N/A71Q (SEQ ID NO: 99), E35N/A71T (SEQ ID NO: 100), E35S/A71E (SEQ ID NO: 101), E35S/A71N (SEQ ID NO: 102), E35S/A71Q (SEQ ID NO: 103), E35S/A71T (SEQ ID NO: 104), E35Q/A71E (SEQ ID NO: 105), E35Q/A71N (SEQ ID NO: 106), E35Q/A71Q (SEQ ID NO: 107), E35Q/A71T (SEQ ID NO: 108), E35T/A71E (SEQ ID NO: 109), E35T/A71N (SEQ ID NO: 110), E35T/A71Q (SEQ ID NO: 112), E35T/A71T (SEQ ID NO: 113), M47A/I69Y/A71E/V83L (SEQ ID NO: 114), M47A/I69W/A71E/V83L (SEQ ID NO: 115), M47A/I69Y/A71E (SEQ ID NO: 116), M47A/I69W/A71E (SEQ ID NO: 117), I69Y/A71E/V83L (SEQ ID NO: 118), I69W/A71E/V83L (SEQ ID NO: 119), I69Y/A71E (SEQ ID NO: 120), I69W/A71E (SEQ ID NO: 121), H18W/A26M/A71E/L85N (SEQ ID NO: 122), H18W/A26M/A71E/L85D (SEQ ID NO: 123), H18W/A26C/A71E/L85N (SEQ ID NO: 124), H18W/A26C/A71E/L85D (SEQ ID NO: 125), A26M/A71E/L85N (SEQ ID NO: 126), A26M/A71E/L85D (SEQ ID NO: 127), A26C/A71E/L85D (SEQ ID NO: 128), A26C/A71E/L85N (SEQ ID NO: 129), I69L/A71E/V83L (SEQ ID NO: 130), H18W/A26V/E35N/A71E/L85N (SEQ ID NO: 131), T13K/M42L/M47H/A71E (SEQ ID NO: 132), T13K/M42L/M47Q/A71E (SEQ ID NO: 133), T13K/M42L/A71E (SEQ ID NO: 134), T13K/M42T/M47H/A71E (SEQ ID NO: 135), T13K/M42T/M47T/A71E (SEQ ID NO: 136), T13K/M42T/A71E (SEQ ID NO: 136), M47H/I69Y/A71E/V83L (SEQ ID NO: 137), M47Q/I69Y/A71E/V83L (SEQ ID NO: 138) or M47K/I69Y/A71E/V83L (SEQ ID NO: 139); or
wherein the first polypeptide is selected from E35N/A71E (SEQ ID NO: 142), E35N/A71N (SEQ ID NO: 143), E35N/A71Q (SEQ ID NO: 144), E35N/A71T (SEQ ID NO: 145), E35S/A71E (SEQ ID NO: 146), E35S/A71N (SEQ ID NO: 147), E35S/A71Q (SEQ ID NO: 148), E35S/A71T (SEQ ID NO: 149), E35Q/A71E (SEQ ID NO: 150), E35Q/A71N (SEQ ID NO: 151), E35Q/A71Q (SEQ ID NO: 152), E35Q/A71T (SEQ ID NO: 153), E35T/A71E (SEQ ID NO: 154), E35T/A71N (SEQ ID NO: 155), E35T/A71Q (SEQ ID NO: 156) or E35T/A71T (SEQ ID NO: 157); or
wherein the first polypeptide is selected from E35N/A71E (SEQ ID NO: 192), E35N/A71N (SEQ ID NO: 193), E35N/A71Q (SEQ ID NO: 194) or E35N/A71T (SEQ ID NO: 195); or
wherein the first polypeptide is selected from E35N/A71E (SEQ ID NO: 198), E35N/A71N (SEQ ID NO: 199), E35N/A71Q (SEQ ID NO: 200) or E35N/A71T (SEQ ID NO: 201); or
wherein the first polypeptide is selected from E35N/A71E (SEQ ID NO: 203), E35N/A71N (SEQ ID NO: 204), E35N/A71Q (SEQ ID NO: 205) or E35N/A71T (SEQ ID NO: 206); or
wherein the first polypeptide is selected from E35N/A71E (SEQ ID NO: 209), E35N/A71N (SEQ ID NO: 210), E35N/A71Q (SEQ ID NO: 211) or E35N/A71T (SEQ ID NO: 212).

17. The CD80 mutant fusion polypeptide complex of claim 15 or 16, wherein the second polypeptide is selected from SEQ ID NO: 141, SEQ ID NO: 197 or SEQ ID NO: 208.

18. The CD80 mutant fusion polypeptide complex of any one of claims 5 to 17, further comprising a third domain, which is covalently linked to the CD80 IgV mutant polypeptide of claim 1 or 2, or the CD80 ECD mutant polypeptide of claim 3 or 4, or the second domain of any one of claims 5 to 17, with or without a linker peptide.

19. The CD80 mutant fusion polypeptide complex of claim 18, wherein the third domain is an antibody or antigen-binding fragment that is the same as or different from the second domain, or the third domain is a functional protein or an active fragment thereof, optionally, when the third domain is a functional protein or an active fragment thereof, the functional protein or an active fragment thereof can activate an immune response, optionally, wherein the third domain is linked to the CD80 IgV mutant polypeptideof claim 1 or 2 or the CD80 ECD mutant polypeptide of claim 3 or 4 with or without a linker peptide.

20. The CD80 mutant fusion polypeptide complex of claim 19, wherein the second domain comprises an antibody heavy chain, and the antibody heavy chain of the second domain is linked to the third domain with or without a linker peptide, and optionally,
wherein, the heavy chain of the second domain is linked to the N-terminus of the third domain with or without a linker peptide, and/or the heavy chain of the second domain is linked to the C-terminus of the third domain with or without a linker peptide; and/or
the second domain comprises an antibody light chain, and the antibody light chain of the second domain is linked to the third domain with or without a linker peptide, optionally, wherein the light chain of the second domain is linked to the N-terminus of the third domain with or without a linker peptide, and/or the light chain of the second domain is linked to the C-terminus of the third domain with or without a linker peptide.

21. The CD80 mutant fusion polypeptide complex of any one of claims 18 to 20, wherein the CD80 mutant fusion polypeptide complex comprises a first polypeptide and a second polypeptide, the second polypeptide may be present or absent, and optionally,
wherein the first polypeptide comprises a heavy chain of the second domain, optionally,
wherein the first polypeptide is covalently linked in sequence from the N-terminus to the C-terminus by a CD80 IgV mutant polypeptide or a CD80 ECD mutant polypeptide, a linker peptide, a heavy chain of an antibody or an antigen-binding fragment thereof, a linker peptide, and a third domain, wherein the linker peptides may be the same or different and may exist or not exist independently; or
wherein, the first polypeptide is covalently linked in sequence from the C-terminus to the N-terminus by a CD80 IgV mutant polypeptide or a CD80 ECD mutant polypeptide, a linker peptide, a heavy chain of an antibody or an antigen-binding fragment thereof, a linker peptide, and a third domain, wherein the linker peptides may be the same or different, and may exist or not exist independently, and/or
wherein the second polypeptide comprises a light chain of a second domain, optionally, wherein the second polypeptide is covalently linked in sequence from the N-terminus to the C-terminus by a CD80 IgV mutant polypeptide or a CD80 ECD mutant polypeptide, a linker peptide, a heavy chain of an antibody or an antigen-binding fragment thereof, a linker peptide, and a third domain, wherein the linker peptides may be the same or different and may exist or not exist independently; or
wherein the second polypeptide is covalently linked in sequence from the C-terminus to the N-terminus by a CD80 IgV mutant polypeptide or a CD80 ECD mutant polypeptide, a linker peptide, a heavy chain of an antibody or an antigen-binding fragment thereof, a linker peptide, and a third domain, wherein the linker peptides may be the same or different and may exist or not exist independently of each other.

22. The CD80 mutant fusion polypeptide complex of any one of claims 18 to 21, wherein the third domain comprises LAG-3 or an active fragment thereof.

23. The CD80 mutant fusion polypeptide complex of claim 22, wherein the second domain specifically targets PD-1 or PD-L1, and optionally, wherein the second domain is sugemalimab or a functional fragment thereof.

24. The CD80 mutant fusion polypeptide complex of claim 22, wherein the first polypeptide comprises a sequence selected from the group consisting of: SEQ ID NO: 293, SEQ ID NO: 295; or the second polypeptide comprises a sequence selected from the group consisting of: SEQ ID NO: 141.

25. The CD80 mutant fusion polypeptide complex of claim 5, wherein the second domain is an immunoglobulin Fc domain, optionally wherein the Fc domain is selected from human IgG1 Fc domain, human IgG2 Fc domain, human IgG3 Fc domain, human IgG4 Fc domain, mouse IgG1 Fc domain, mouse IgG2a Fc domain, mouse IgG2b Fc domain or mouse IgG3 Fc domain, optionally wherein the CD80 IgV mutant polypeptide or CD80 ECD mutant polypeptide is covalently linked to the N-terminus of the immunoglobulin Fc domain with or without a linker peptide.

26. The CD80 mutant fusion polypeptide complex of claim 25, wherein the amino acid sequence of the CD80 mutant fusion polypeptide complex is selected from E35N/A71E (SEQ ID NO: 158), E35N/A71N (SEQ ID NO: 159), E35N/A71Q (SEQ ID NO: 160), E35N/A71T (SEQ ID NO: 161), E35S/A71E (SEQ ID NO: 162), E35S/A71N (SEQ ID NO: 163), E35S/A71Q (SEQ ID NO: 164), E35S/A71T (SEQ ID NO: 165), E35Q/A71E (SEQ ID NO: 166), E35Q/A71N (SEQ ID NO: 167), E35Q/A71Q (SEQ ID NO: 168), E35Q/A71T (SEQ ID NO: 169), E35T/A71E (SEQ ID NO: 170), E35T/A71N (SEQ ID NO: 171), E35T/A71Q (SEQ ID NO: 172) or E35T/A71T (SEQ ID NO: 173).

27. The CD80 mutant fusion polypeptide complex of claim 25 or 26, wherein the C-terminus of the immunoglobulin Fc domain is covalently linked to the CD80 IgV mutant polypeptide or CD80 ECD mutant polypeptide with or without a linker peptide.

28. The CD80 mutant fusion polypeptide complex of any one of claims 5 to 27, wherein when a linker peptide is present, the linker peptide is selected from one or more of GGGGS (SEQ ID NO: 92), GGGGSGGGGS (SEQ ID NO: 93), GGGGSGGGSGGGGGS (SEQ ID NO: 94),GGGGSGGGGSGGGGSGGGGS (SEQ ID NO: 95) and GGGGSGGGS (SEQ ID NO: 96).

29. An immunoconjugate comprising the CD80 IgV mutant polypeptide of claim 1 or 2, the CD80 ECD mutant polypeptide of claim 3 or 4, or the CD80 mutant fusion polypeptide complex of any one of claims 5-28.

30. A nucleotide molecule encoding the CD80 IgV mutant polypeptide of claim 1 or 2, the CD80 ECD mutant polypeptide of claim 3 or 4, or the CD80 mutant fusion polypeptide complex of any one of claims 5-28.

31. A vector comprising the nucleotide molecule of claim 30.

32. A host cell comprising the CD80 IgV mutant polypeptide of claim 1 or 2, the CD80 ECD mutant polypeptide of claim 3 or 4, the CD80 mutant fusion polypeptide complex of any one of claims 5-28, the nucleotide molecule of claim 30 and/or the vector of claim 31.

33. A composition comprising the CD80 IgV mutant polypeptide of claim 1 or 2, the CD80 ECD mutant polypeptide of claim 3 or 4, the CD80 mutant fusion polypeptide complex of any one of claims 5-28, the immunoconjugate of claim 29, the nucleotide molecule of claim 30 or the vector of claim 31 and a pharmaceutically acceptable carrier.

34. A method for preparing the CD80 IgV mutant polypeptide of claim 1 or 2, the CD80 ECD mutant polypeptide of claim 3 or 4, or the CD80 mutant fusion polypeptide complex of any one of claims 5-28, comprising culturing the host cell of claim 32 to express the CD80 IgV mutant polypeptide, the CD80 ECD mutant polypeptide and/or the CD80 mutant fusion polypeptide complex.

35. A method for regulating T cell activity, comprising contacting the CD80 IgV mutant polypeptide of claim 1 or 2, the CD80 ECD mutant polypeptide of claim 3 or 4, or the CD80 mutant fusion polypeptide complex of any one of claims 5 to 28 and/or the composition of claim 33 with a T cell to stimulate its activation.

36. A method for inhibiting tumor cell growth or proliferation, comprising administering the CD80 IgV mutant polypeptide of claim 1 or 2, the CD80 ECD mutant polypeptide of claim 3 or 4, the CD80 mutant fusion polypeptide complex of any one of claims 5-28, the immunoconjugate of claim 29, the nucleotide molecule of claim 30, the vector of claim 31, the host cell of claim 32 and/or the composition of claim 33.

37. Use of the CD80 IgV mutant polypeptide of claim 1 or 2, the CD80 ECD mutant polypeptide of claim 3 or 4, the CD80 mutant fusion polypeptide complex of any one of claims 5-28, the immunoconjugate of claim 29, the nucleotide molecule of claim 30, the vector of claim 31, the host cell of claim 32 and/or the composition of claim 33 in the preparation of a medicament for preventing, improving and/or treating tumors or cancer.
